# EUROPEAN PATENT APPLICATION

(11) **EP 3 594 229 A1**
(43) Date of publication of application: **15.01.2020**
(21) Application number: 18382516.5
(22) Date of filing: 11.07.2018
(51) Int. Cl.: C07K 14/78, C12N 9/50

(54) **RECOMBINANT BIOPOLYMER FOR PROTEASES DETECTION**

(71) Applicant: Universidad De Valladolid, 47002 Valladolid (ES)
(72) Inventor: RODRÍGUEZ CABELLO, José Carlos, 47011 Valladolid (ES); ALONSO RODRIGO, Matilde, 47011 Valladolid (ES); POOCZA, Leander, 47011 Valladolid (ES)
(74) Representative: Pons

(57) **Abstract**

The present invention relates to a biopolymer, bioactive and totally biocompatible, comprising three different building regions, which can be repeated several times for higher specificity, wherein one region comprises a bioactive domain capable of identify proteolytic activity in a precise manner and the additional two regions are based on elastin-mimetic proteins. Furthermore, the invention relates to any of the uses thereof, and a method of synthesis thereof.

SEQUENCE LISTING

<110>  Universidad de Valladolid

<120>  RECOMBINANT BIOPOLYMER FOR PROTEASES DETECTION

<130>  EP2080.19

<160>  38

<170>  PatentIn version 3.5

<210>  1
<211>  5
<212>  PRT
<213>  Artificial Sequence

<220>
<223>  elastin-like protein domain

<220>
<221>  misc_feature
<222>  (4)..(4)
<223>  Xaa can be any naturally occurring amino acid besides proline

<400>  1

Val Pro Gly Xaa Gly
1               5

<210>  2
<211>  5
<212>  PRT
<213>  Artificial Sequence

<220>
<223>  Elastin-like protein domain

<400>  2

Val Pro Gly Ile Gly
1               5

<210>  3
<211>  5
<212>  PRT
<213>  Artificial Sequence

<220>
<223>  Elastin-like protein domain

<400>  3

Val Pro Gly Val Gly
1               5

<210>  4
<211>  5
<212>  PRT

<213>  Artificial Sequence

<220>
<223>  Elastin-like protein domain

<400>  4

Val Pro Gly Glu Gly
1               5


<210>  5
<211>  5
<212>  PRT
<213>  Artificial Sequence

<220>
<223>  Elastin-like protein domain


<220>
<221>  misc_feature
<222>  (4)..(4)
<223>  Xaa can be a naturally occurring amino acid selected from any of
       the followings: lysine, arginine or histidine

<400>  5

Val Pro Gly Xaa Gly
1               5


<210>  6
<211>  5
<212>  PRT
<213>  Artificial Sequence

<220>
<223>  Elastin-like protein domain

<400>  6

Val Pro Gly Lys Gly
1               5


<210>  7
<211>  3
<212>  PRT
<213>  Artificial Sequence

<220>
<223>  Block B-Staining region

<400>  7

Lys Lys Lys
1


<210>  8
<211>  13
<212>  PRT

<213>  Artificial Sequence

<220>
<223>  Block B-Staining region

<400>  8

Gly Gly Gly Gly Gly Lys Lys Lys Gly Gly Gly Gly Val
1               5                   10


<210>  9
<211>  18
<212>  PRT
<213>  Artificial Sequence

<220>
<223>  Elastase specific proteolytic domain

<400>  9

Val Gly Val Ala Pro Gly Val Gly Val Ala Pro Gly Val Gly Val Ala
1               5                   10                  15


Pro Gly




<210>  10
<211>  17
<212>  PRT
<213>  Artificial Sequence

<220>
<223>  Urokinase-type Plasminogen Activator (u-PA) specific proteolytic
       domain

<400>  10

Tyr Ala Val Thr Gly Asp Arg Ile Arg Ser Ala Ser Pro Ala Ser Ser
1               5                   10                  15


Ala




<210>  11
<211>  17
<212>  PRT
<213>  Artificial Sequence

<220>
<223>  Urokinase-type Plasminogen Activator (u-PA) specific proteolytic
       domain

<400>  11

Tyr Ala Val Thr Gly Gly Thr Ala Arg Ser Ala Ser Pro Ala Ser Ser
1               5                   10                  15

Ala

<210>  12
<211>  17
<212>  PRT
<213>  Artificial Sequence

<220>
<223>  Tissue-type Plasminogen Activator (t-PA) specific proteolytic
       domain

<400>  12

Tyr Ala Val Thr Gly Asp Asn Arg Arg Ser Ala Ser Pro Ala Ser Ser
1               5                   10                  15

Ala

<210>  13
<211>  17
<212>  PRT
<213>  Artificial Sequence

<220>
<223>  Tissue-type Plasminogen Activator (t-PA) specific proteolytic
       domain

<400>  13

Tyr Ala Val Thr Gly Phe Phe Ser Arg Ser Ala Ser Pro Ala Ser Ser
1               5                   10                  15

Ala

<210>  14
<211>  51
<212>  PRT
<213>  Artificial Sequence

<220>
<223>  MMP-2 specific proteolytic domain

<400>  14

Val Gly Gly Gly Gly Gly Pro Met Gly Pro Ser Gly Pro Trp Gly Gly
1               5                   10                  15

Gly Gly Gly Val Gly Gly Gly Gly Gln Pro Gln Gly Leu Ala Lys Gly
            20                  25                  30

Gly Gly Val Gly Gly Gly Gly Gly Pro Gln Gly Ile Trp Gly Gln Gly
        35                  40                  45

Gly Gly Gly
    50

<210>  15
<211>  12
<212>  PRT
<213>  Artificial Sequence

<220>
<223>  Integrin specific binding domain

<400>  15

Ala Val Thr Gly Arg Gly Asp Ser Pro Ala Ser Ser
1               5                   10

<210>  16
<211>  19
<212>  PRT
<213>  Artificial Sequence

<220>
<223>  Integrin specific binding domain

<400>  16

Glu Glu Ile Gln Ile Gly His Ile Pro Arg Glu Asp Val Asp Tyr His
1               5                   10                  15

Leu Tyr Pro

<210>  17
<211>  4
<212>  PRT
<213>  Artificial Sequence

<220>
<223>  Elastin-like protein domain

<400>  17

Val Pro Gly Gly
1

<210>  18
<211>  680
<212>  PRT
<213>  Artificial Sequence

<220>
<223>  Biopolymer

<400>  18

Val Pro Gly Ile Gly Val Pro Gly Ile Gly Val Pro Gly Lys Gly Val
1               5                   10                  15

Pro Gly Ile Gly Val Pro Gly Ile Gly Val Pro Gly Ile Gly Val Pro
            20                  25                  30

Gly Ile Gly Val Pro Gly Lys Gly Val Pro Gly Ile Gly Val Pro Gly
        35                  40                  45

Ile Gly Val Gly Val Ala Pro Gly Val Gly Val Ala Pro Gly Val Gly
    50                  55                  60

Val Ala Pro Gly Val Pro Gly Ile Gly Val Pro Gly Ile Gly Val Pro
65                  70                  75                  80

Gly Lys Gly Val Pro Gly Ile Gly Val Pro Gly Ile Gly Val Pro Gly
                85                  90                  95

Ile Gly Val Pro Gly Ile Gly Val Pro Gly Lys Gly Val Pro Gly Ile
            100                 105                 110

Gly Val Pro Gly Ile Gly Val Gly Val Ala Pro Gly Val Gly Val Ala
        115                 120                 125

Pro Gly Val Gly Val Ala Pro Gly Val Pro Gly Ile Gly Val Pro Gly
    130                 135                 140

Ile Gly Val Pro Gly Lys Gly Val Pro Gly Ile Gly Val Pro Gly Ile
145                 150                 155                 160

Gly Val Pro Gly Ile Gly Val Pro Gly Ile Gly Val Pro Gly Lys Gly
                165                 170                 175

Val Pro Gly Ile Gly Val Pro Gly Ile Gly Val Gly Val Ala Pro Gly
            180                 185                 190

Val Gly Val Ala Pro Gly Val Gly Val Ala Pro Gly Val Pro Gly Ile
        195                 200                 205

Gly Val Pro Gly Ile Gly Val Pro Gly Lys Gly Val Pro Gly Ile Gly
    210                 215                 220

Val Pro Gly Ile Gly Val Pro Gly Ile Gly Val Pro Gly Ile Gly Val
225                 230                 235                 240

Pro Gly Lys Gly Val Pro Gly Ile Gly Val Pro Gly Ile Gly Val Gly
                245                 250                 255

Val Ala Pro Gly Val Gly Val Ala Pro Gly Val Gly Val Ala Pro Gly

260                 265                 270

Val Pro Gly Ile Gly Val Pro Gly Ile Gly Val Pro Gly Lys Gly Val
        275                 280                 285

Pro Gly Ile Gly Val Pro Gly Ile Gly Val Pro Gly Ile Gly Val Pro
    290                 295                 300

Gly Ile Gly Val Pro Gly Lys Gly Val Pro Gly Ile Gly Val Pro Gly
305                 310                 315                 320

Ile Gly Val Gly Val Ala Pro Gly Val Gly Val Ala Pro Gly Val Gly
                325                 330                 335

Val Ala Pro Gly Val Pro Gly Ile Gly Val Pro Gly Ile Gly Val Pro
            340                 345                 350

Gly Lys Gly Val Pro Gly Ile Gly Val Pro Gly Ile Gly Val Pro Gly
        355                 360                 365

Ile Gly Val Pro Gly Ile Gly Val Pro Gly Lys Gly Val Pro Gly Ile
    370                 375                 380

Gly Val Pro Gly Ile Gly Val Gly Val Ala Pro Gly Val Gly Val Ala
385                 390                 395                 400

Pro Gly Val Gly Val Ala Pro Gly Val Pro Gly Ile Gly Val Pro Gly
                405                 410                 415

Ile Gly Val Pro Gly Lys Gly Val Pro Gly Ile Gly Val Pro Gly Ile
            420                 425                 430

Gly Val Pro Gly Ile Gly Val Pro Gly Ile Gly Val Pro Gly Lys Gly
        435                 440                 445

Val Pro Gly Ile Gly Val Pro Gly Ile Gly Val Gly Val Ala Pro Gly
    450                 455                 460

Val Gly Val Ala Pro Gly Val Gly Val Ala Pro Gly Val Pro Gly Ile
465                 470                 475                 480

Gly Val Pro Gly Ile Gly Val Pro Gly Lys Gly Val Pro Gly Ile Gly
                485                 490                 495

Val Pro Gly Ile Gly Val Pro Gly Ile Gly Val Pro Gly Ile Gly Val
            500                 505                 510

Pro Gly Lys Gly Val Pro Gly Ile Gly Val Pro Gly Ile Gly Val Gly
        515                 520                 525

Val Ala Pro Gly Val Gly Val Ala Pro Gly Val Gly Val Ala Pro Gly
    530                 535                 540

Val Pro Gly Ile Gly Val Pro Gly Ile Gly Val Pro Gly Lys Gly Val
545                 550                 555                 560

Pro Gly Ile Gly Val Pro Gly Ile Gly Val Pro Gly Ile Gly Val Pro
                565                 570                 575

Gly Ile Gly Val Pro Gly Lys Gly Val Pro Gly Ile Gly Val Pro Gly
            580                 585                 590

Ile Gly Val Gly Val Ala Pro Gly Val Gly Val Ala Pro Gly Val Gly
        595                 600                 605

Val Ala Pro Gly Val Pro Gly Ile Gly Val Pro Gly Ile Gly Val Pro
    610                 615                 620

Gly Lys Gly Val Pro Gly Ile Gly Val Pro Gly Ile Gly Val Pro Gly
625                 630                 635                 640

Ile Gly Val Pro Gly Ile Gly Val Pro Gly Lys Gly Val Pro Gly Ile
                645                 650                 655

Gly Val Pro Gly Ile Gly Val Gly Val Ala Pro Gly Val Gly Val Ala
            660                 665                 670

Pro Gly Val Gly Val Ala Pro Gly
        675                 680

<210>  19
<211>  472
<212>  PRT
<213>  Artificial Sequence

<220>
<223>  Biopolymer

<400>  19

Val Pro Gly Ile Gly Val Pro Gly Ile Gly Val Pro Gly Lys Gly Val
1               5                   10                  15

Pro Gly Ile Gly Val Pro Gly Ile Gly Val Pro Gly Ile Gly Val Pro
            20                  25                  30

Gly Ile Gly Val Pro Gly Lys Gly Val Pro Gly Ile Gly Val Pro Gly

35                  40                  45

Ile Gly Val Tyr Ala Val Thr Gly Asp Arg Ile Arg Ser Ala Ser Pro
    50                  55                  60

Ala Ser Ser Ala Val Pro Gly Ile Gly Val Pro Gly Ile Gly Val Pro
65                  70                  75                  80

Gly Lys Gly Val Pro Gly Ile Gly Val Pro Gly Ile Gly Val Pro Gly
                85                  90                  95

Ile Gly Val Pro Gly Ile Gly Val Pro Gly Lys Gly Val Pro Gly Ile
            100                 105                 110

Gly Val Pro Gly Ile Gly Val Pro Gly Ile Gly Val Pro Gly Ile Gly
        115                 120                 125

Val Pro Gly Lys Gly Val Pro Gly Ile Gly Val Pro Gly Ile Gly Val
    130                 135                 140

Pro Gly Ile Gly Val Pro Gly Ile Gly Val Pro Gly Lys Gly Val Pro
145                 150                 155                 160

Gly Ile Gly Val Pro Gly Ile Gly Val Tyr Ala Val Thr Gly Asp Arg
                165                 170                 175

Ile Arg Ser Ala Ser Pro Ala Ser Ser Ala Val Pro Gly Ile Gly Val
            180                 185                 190

Pro Gly Ile Gly Val Pro Gly Lys Gly Val Pro Gly Ile Gly Val Pro
        195                 200                 205

Gly Ile Gly Val Pro Gly Ile Gly Val Pro Gly Ile Gly Val Pro Gly
    210                 215                 220

Lys Gly Val Pro Gly Ile Gly Val Pro Gly Ile Gly Val Pro Gly Ile
225                 230                 235                 240

Gly Val Pro Gly Ile Gly Val Pro Gly Lys Gly Val Pro Gly Ile Gly
                245                 250                 255

Val Pro Gly Ile Gly Val Pro Gly Ile Gly Val Pro Gly Ile Gly Val
            260                 265                 270

Pro Gly Lys Gly Val Pro Gly Ile Gly Val Pro Gly Ile Gly Val Tyr
        275                 280                 285

Ala Val Thr Gly Asp Arg Ile Arg Ser Ala Ser Pro Ala Ser Ser Ala
    290                 295                 300

Val Pro Gly Ile Gly Val Pro Gly Ile Gly Val Pro Gly Lys Gly Val
305                 310                 315                 320

Pro Gly Ile Gly Val Pro Gly Ile Gly Val Pro Gly Ile Gly Val Pro
                325                 330                 335

Gly Ile Gly Val Pro Gly Lys Gly Val Pro Gly Ile Gly Val Pro Gly
            340                 345                 350

Ile Gly Val Pro Gly Ile Gly Val Pro Gly Ile Gly Val Pro Gly Lys
        355                 360                 365

Gly Val Pro Gly Ile Gly Val Pro Gly Ile Gly Val Pro Gly Ile Gly
    370                 375                 380

Val Pro Gly Ile Gly Val Pro Gly Lys Gly Val Pro Gly Ile Gly Val
385                 390                 395                 400

Pro Gly Ile Gly Val Tyr Ala Val Thr Gly Asp Arg Ile Arg Ser Ala
                405                 410                 415

Ser Pro Ala Ser Ser Ala Val Pro Gly Ile Gly Val Pro Gly Ile Gly
            420                 425                 430

Val Pro Gly Lys Gly Val Pro Gly Ile Gly Val Pro Gly Ile Gly Val
        435                 440                 445

Pro Gly Ile Gly Val Pro Gly Ile Gly Val Pro Gly Lys Gly Val Pro
    450                 455                 460

Gly Ile Gly Val Pro Gly Ile Gly
465                 470

<210>  20
<211>  472
<212>  PRT
<213>  Artificial Sequence

<220>
<223>  Biopolymer

<400>  20

Val Pro Gly Ile Gly Val Pro Gly Ile Gly Val Pro Gly Lys Gly Val
1               5                   10                  15

Pro Gly Ile Gly Val Pro Gly Ile Gly Val Pro Gly Ile Gly Val Pro

20                  25                  30

Gly Ile Gly Val Pro Gly Lys Gly Val Pro Gly Ile Gly Val Pro Gly
        35                  40                  45

Ile Gly Val Tyr Ala Val Thr Gly Gly Thr Ala Arg Ser Ala Ser Pro
    50                  55                  60

Ala Ser Ser Ala Val Pro Gly Ile Gly Val Pro Gly Ile Gly Val Pro
65                  70                  75                  80

Gly Lys Gly Val Pro Gly Ile Gly Val Pro Gly Ile Gly Val Pro Gly
                85                  90                  95

Ile Gly Val Pro Gly Ile Gly Val Pro Gly Lys Gly Val Pro Gly Ile
            100                 105                 110

Gly Val Pro Gly Ile Gly Val Pro Gly Ile Gly Val Pro Gly Ile Gly
        115                 120                 125

Val Pro Gly Lys Gly Val Pro Gly Ile Gly Val Pro Gly Ile Gly Val
    130                 135                 140

Pro Gly Ile Gly Val Pro Gly Ile Gly Val Pro Gly Lys Gly Val Pro
145                 150                 155                 160

Gly Ile Gly Val Pro Gly Ile Gly Val Tyr Ala Val Thr Gly Gly Thr
                165                 170                 175

Ala Arg Ser Ala Ser Pro Ala Ser Ser Ala Val Pro Gly Ile Gly Val
            180                 185                 190

Pro Gly Ile Gly Val Pro Gly Lys Gly Val Pro Gly Ile Gly Val Pro
        195                 200                 205

Gly Ile Gly Val Pro Gly Ile Gly Val Pro Gly Ile Gly Val Pro Gly
    210                 215                 220

Lys Gly Val Pro Gly Ile Gly Val Pro Gly Ile Gly Val Pro Gly Ile
225                 230                 235                 240

Gly Val Pro Gly Ile Gly Val Pro Gly Lys Gly Val Pro Gly Ile Gly
                245                 250                 255

Val Pro Gly Ile Gly Val Pro Gly Ile Gly Val Pro Gly Ile Gly Val
            260                 265                 270

Pro Gly Lys Gly Val Pro Gly Ile Gly Val Pro Gly Ile Gly Val Tyr
        275                 280                 285

Ala Val Thr Gly Gly Thr Ala Arg Ser Ala Ser Pro Ala Ser Ser Ala
    290                 295                 300

Val Pro Gly Ile Gly Val Pro Gly Ile Gly Val Pro Gly Lys Gly Val
305                 310                 315                 320

Pro Gly Ile Gly Val Pro Gly Ile Gly Val Pro Gly Ile Gly Val Pro
                325                 330                 335

Gly Ile Gly Val Pro Gly Lys Gly Val Pro Gly Ile Gly Val Pro Gly
            340                 345                 350

Ile Gly Val Pro Gly Ile Gly Val Pro Gly Ile Gly Val Pro Gly Lys
        355                 360                 365

Gly Val Pro Gly Ile Gly Val Pro Gly Ile Gly Val Pro Gly Ile Gly
    370                 375                 380

Val Pro Gly Ile Gly Val Pro Gly Lys Gly Val Pro Gly Ile Gly Val
385                 390                 395                 400

Pro Gly Ile Gly Val Tyr Ala Val Thr Gly Gly Thr Ala Arg Ser Ala
                405                 410                 415

Ser Pro Ala Ser Ser Ala Val Pro Gly Ile Gly Val Pro Gly Ile Gly
            420                 425                 430

Val Pro Gly Lys Gly Val Pro Gly Ile Gly Val Pro Gly Ile Gly Val
        435                 440                 445

Pro Gly Ile Gly Val Pro Gly Ile Gly Val Pro Gly Lys Gly Val Pro
    450                 455                 460

Gly Ile Gly Val Pro Gly Ile Gly
465                 470


<210>  21
<211>  472
<212>  PRT
<213>  Artificial Sequence

<220>
<223>  Biopolymer

<400>  21

Val Pro Gly Ile Gly Val Pro Gly Ile Gly Val Pro Gly Lys Gly Val

1               5                   10                  15

Pro Gly Ile Gly Val Pro Gly Ile Gly Val Pro Gly Ile Gly Val Pro
            20                  25                  30

Gly Ile Gly Val Pro Gly Lys Gly Val Pro Gly Ile Gly Val Pro Gly
        35                  40                  45

Ile Gly Val Tyr Ala Val Thr Gly Asp Asn Arg Arg Ser Ala Ser Pro
    50                  55                  60

Ala Ser Ser Ala Val Pro Gly Ile Gly Val Pro Gly Ile Gly Val Pro
65                  70                  75                  80

Gly Lys Gly Val Pro Gly Ile Gly Val Pro Gly Ile Gly Val Pro Gly
                85                  90                  95

Ile Gly Val Pro Gly Ile Gly Val Pro Gly Lys Gly Val Pro Gly Ile
            100                 105                 110

Gly Val Pro Gly Ile Gly Val Pro Gly Ile Gly Val Pro Gly Ile Gly
        115                 120                 125

Val Pro Gly Lys Gly Val Pro Gly Ile Gly Val Pro Gly Ile Gly Val
    130                 135                 140

Pro Gly Ile Gly Val Pro Gly Ile Gly Val Pro Gly Lys Gly Val Pro
145                 150                 155                 160

Gly Ile Gly Val Pro Gly Ile Gly Val Tyr Ala Val Thr Gly Asp Asn
                165                 170                 175

Arg Arg Ser Ala Ser Pro Ala Ser Ser Ala Val Pro Gly Ile Gly Val
            180                 185                 190

Pro Gly Ile Gly Val Pro Gly Lys Gly Val Pro Gly Ile Gly Val Pro
        195                 200                 205

Gly Ile Gly Val Pro Gly Ile Gly Val Pro Gly Ile Gly Val Pro Gly
    210                 215                 220

Lys Gly Val Pro Gly Ile Gly Val Pro Gly Ile Gly Val Pro Gly Ile
225                 230                 235                 240

Gly Val Pro Gly Ile Gly Val Pro Gly Lys Gly Val Pro Gly Ile Gly
                245                 250                 255

Val Pro Gly Ile Gly Val Pro Gly Ile Gly Val Pro Gly Ile Gly Val
        260                 265                 270

Pro Gly Lys Gly Val Pro Gly Ile Gly Val Pro Gly Ile Gly Val Tyr
    275                 280                 285

Ala Val Thr Gly Asp Asn Arg Arg Ser Ala Ser Pro Ala Ser Ser Ala
    290                 295                 300

Val Pro Gly Ile Gly Val Pro Gly Ile Gly Val Pro Gly Lys Gly Val
305                 310                 315                 320

Pro Gly Ile Gly Val Pro Gly Ile Gly Val Pro Gly Ile Gly Val Pro
                325                 330                 335

Gly Ile Gly Val Pro Gly Lys Gly Val Pro Gly Ile Gly Val Pro Gly
        340                 345                 350

Ile Gly Val Pro Gly Ile Gly Val Pro Gly Ile Gly Val Pro Gly Lys
    355                 360                 365

Gly Val Pro Gly Ile Gly Val Pro Gly Ile Gly Val Pro Gly Ile Gly
    370                 375                 380

Val Pro Gly Ile Gly Val Pro Gly Lys Gly Val Pro Gly Ile Gly Val
385                 390                 395                 400

Pro Gly Ile Gly Val Tyr Ala Val Thr Gly Asp Asn Arg Arg Ser Ala
                405                 410                 415

Ser Pro Ala Ser Ser Ala Val Pro Gly Ile Gly Val Pro Gly Ile Gly
        420                 425                 430

Val Pro Gly Lys Gly Val Pro Gly Ile Gly Val Pro Gly Ile Gly Val
    435                 440                 445

Pro Gly Ile Gly Val Pro Gly Ile Gly Val Pro Gly Lys Gly Val Pro
    450                 455                 460

Gly Ile Gly Val Pro Gly Ile Gly
465                 470

<210>  22
<211>  472
<212>  PRT
<213>  Artificial Sequence

<220>
<223>  Biopolymer

<400>  22

Val Pro Gly Ile Gly Val Pro Gly Ile Gly Val Pro Gly Lys Gly Val
1               5                   10                  15

Pro Gly Ile Gly Val Pro Gly Ile Gly Val Pro Gly Ile Gly Val Pro
            20                  25                  30

Gly Ile Gly Val Pro Gly Lys Gly Val Pro Gly Ile Gly Val Pro Gly
        35                  40                  45

Ile Gly Val Tyr Ala Val Thr Gly Phe Phe Ser Arg Ser Ala Ser Pro
    50                  55                  60

Ala Ser Ser Ala Val Pro Gly Ile Gly Val Pro Gly Ile Gly Val Pro
65                  70                  75                  80

Gly Lys Gly Val Pro Gly Ile Gly Val Pro Gly Ile Gly Val Pro Gly
                85                  90                  95

Ile Gly Val Pro Gly Ile Gly Val Pro Gly Lys Gly Val Pro Gly Ile
            100                 105                 110

Gly Val Pro Gly Ile Gly Val Pro Gly Ile Gly Val Pro Gly Ile Gly
        115                 120                 125

Val Pro Gly Lys Gly Val Pro Gly Ile Gly Val Pro Gly Ile Gly Val
    130                 135                 140

Pro Gly Ile Gly Val Pro Gly Ile Gly Val Pro Gly Lys Gly Val Pro
145                 150                 155                 160

Gly Ile Gly Val Pro Gly Ile Gly Val Tyr Ala Val Thr Gly Phe Phe
                165                 170                 175

Ser Arg Ser Ala Ser Pro Ala Ser Ser Ala Val Pro Gly Ile Gly Val
            180                 185                 190

Pro Gly Ile Gly Val Pro Gly Lys Gly Val Pro Gly Ile Gly Val Pro
        195                 200                 205

Gly Ile Gly Val Pro Gly Ile Gly Val Pro Gly Ile Gly Val Pro Gly
    210                 215                 220

Lys Gly Val Pro Gly Ile Gly Val Pro Gly Ile Gly Val Pro Gly Ile
225                 230                 235                 240

Gly Val Pro Gly Ile Gly Val Pro Gly Lys Gly Val Pro Gly Ile Gly
                245                 250                 255

Val Pro Gly Ile Gly Val Pro Gly Ile Gly Val Pro Gly Ile Gly Val
            260                 265                 270

Pro Gly Lys Gly Val Pro Gly Ile Gly Val Pro Gly Ile Gly Val Tyr
        275                 280                 285

Ala Val Thr Gly Phe Phe Ser Arg Ser Ala Ser Pro Ala Ser Ser Ala
    290                 295                 300

Val Pro Gly Ile Gly Val Pro Gly Ile Gly Val Pro Gly Lys Gly Val
305                 310                 315                 320

Pro Gly Ile Gly Val Pro Gly Ile Gly Val Pro Gly Ile Gly Val Pro
                325                 330                 335

Gly Ile Gly Val Pro Gly Lys Gly Val Pro Gly Ile Gly Val Pro Gly
            340                 345                 350

Ile Gly Val Pro Gly Ile Gly Val Pro Gly Ile Gly Val Pro Gly Lys
        355                 360                 365

Gly Val Pro Gly Ile Gly Val Pro Gly Ile Gly Val Pro Gly Ile Gly
    370                 375                 380

Val Pro Gly Ile Gly Val Pro Gly Lys Gly Val Pro Gly Ile Gly Val
385                 390                 395                 400

Pro Gly Ile Gly Val Tyr Ala Val Thr Gly Phe Phe Ser Arg Ser Ala
                405                 410                 415

Ser Pro Ala Ser Ser Ala Val Pro Gly Ile Gly Val Pro Gly Ile Gly
            420                 425                 430

Val Pro Gly Lys Gly Val Pro Gly Ile Gly Val Pro Gly Ile Gly Val
        435                 440                 445

Pro Gly Ile Gly Val Pro Gly Ile Gly Val Pro Gly Lys Gly Val Pro
    450                 455                 460

Gly Ile Gly Val Pro Gly Ile Gly
465                 470

<210>  23
<211>  632
<212>  PRT

<213>  Artificial Sequence

<220>
<223>  Biopolymer

<400>  23

Lys Lys Lys Pro Val Pro Gly Val Gly Val Pro Gly Val Gly Val Pro
1               5                   10                  15

Gly Glu Gly Val Pro Gly Val Gly Val Pro Gly Val Gly Val Pro Gly
            20                  25                  30

Val Gly Val Pro Gly Val Gly Val Pro Gly Glu Gly Val Pro Gly Val
        35                  40                  45

Gly Val Pro Gly Val Gly Val Pro Gly Val Gly Val Pro Gly Val Gly
    50                  55                  60

Val Pro Gly Glu Gly Val Pro Gly Val Gly Val Pro Gly Val Gly Val
65                  70                  75                  80

Pro Gly Val Gly Val Pro Gly Val Gly Val Pro Gly Glu Gly Val Pro
                85                  90                  95

Gly Val Gly Val Pro Gly Val Gly Val Pro Gly Val Gly Val Pro Gly
            100                 105                 110

Val Gly Val Pro Gly Glu Gly Val Pro Gly Val Gly Val Pro Gly Val
        115                 120                 125

Gly Val Val Pro Gly Val Gly Val Pro Gly Val Gly Val Pro Gly Glu
    130                 135                 140

Gly Val Pro Gly Val Gly Val Pro Gly Val Gly Val Pro Gly Val Gly
145                 150                 155                 160

Val Pro Gly Val Gly Val Pro Gly Glu Gly Val Pro Gly Val Gly Val
                165                 170                 175

Pro Gly Val Gly Val Pro Gly Val Gly Val Pro Gly Val Gly Val Pro
            180                 185                 190

Gly Glu Gly Val Pro Gly Val Gly Val Pro Gly Val Gly Val Pro Gly
        195                 200                 205

Val Gly Val Pro Gly Val Gly Val Pro Gly Glu Gly Val Pro Gly Val
    210                 215                 220

Gly Val Pro Gly Val Gly Val Pro Gly Val Gly Val Pro Gly Val Gly
225                 230                 235                 240

Val Pro Gly Glu Gly Val Pro Gly Val Gly Val Pro Gly Val Gly Val
            245                 250                 255

Val Gly Gly Gly Gly Gly Pro Met Gly Pro Ser Gly Pro Trp Gly Gly
        260                 265                 270

Gly Gly Gly Val Gly Gly Gly Gly Gln Pro Gln Gly Leu Ala Lys Gly
    275                 280                 285

Gly Gly Gly Gly Val Gly Gly Gly Gly Gly Pro Gln Gly Ile Trp Gly
    290                 295                 300

Gln Gly Gly Gly Gly Val Gly Gly Gly Gly Lys Lys Lys Gly Gly Gly
305                 310                 315                 320

Gly Val Val Pro Gly Val Gly Val Pro Gly Val Gly Val Pro Gly Glu
            325                 330                 335

Gly Val Pro Gly Val Gly Val Pro Gly Val Gly Val Pro Gly Val Gly
        340                 345                 350

Val Pro Gly Val Gly Val Pro Gly Glu Gly Val Pro Gly Val Gly Val
    355                 360                 365

Pro Gly Val Gly Val Pro Gly Val Gly Val Pro Gly Val Gly Val Pro
    370                 375                 380

Gly Glu Gly Val Pro Gly Val Gly Val Pro Gly Val Gly Val Pro Gly
385                 390                 395                 400

Val Gly Val Pro Gly Val Gly Val Pro Gly Glu Gly Val Pro Gly Val
            405                 410                 415

Gly Val Pro Gly Val Gly Val Pro Gly Val Gly Val Pro Gly Val Gly
        420                 425                 430

Val Pro Gly Glu Gly Val Pro Gly Val Gly Val Pro Gly Val Gly Val
    435                 440                 445

Val Pro Gly Val Gly Val Pro Gly Val Gly Val Pro Gly Glu Gly Val
    450                 455                 460

Pro Gly Val Gly Val Pro Gly Val Gly Val Pro Gly Val Gly Val Pro
465                 470                 475                 480

Gly Val Gly Val Pro Gly Glu Gly Val Pro Gly Val Gly Val Pro Gly
                    485                 490                 495

Val Gly Val Pro Gly Val Gly Val Pro Gly Val Gly Val Pro Gly Glu
            500                 505                 510

Gly Val Pro Gly Val Gly Val Pro Gly Val Gly Val Pro Gly Val Gly
        515                 520                 525

Val Pro Gly Val Gly Val Pro Gly Glu Gly Val Pro Gly Val Gly Val
    530                 535                 540

Pro Gly Val Gly Val Pro Gly Val Gly Val Pro Gly Val Gly Val Pro
545                 550                 555                 560

Gly Glu Gly Val Pro Gly Val Gly Val Pro Gly Val Gly Val Val Gly
                    565                 570                 575

Gly Gly Gly Gly Pro Met Gly Pro Ser Gly Pro Trp Gly Gly Gly Gly
            580                 585                 590

Gly Val Gly Gly Gly Gly Gln Pro Gln Gly Leu Ala Lys Gly Gly Gly
        595                 600                 605

Gly Gly Val Gly Gly Gly Gly Gly Pro Gln Gly Ile Trp Gly Gln Gly
    610                 615                 620

Gly Gly Gly Val Val Lys Lys Lys
625                 630

<210>  24
<211>  672
<212>  PRT
<213>  Artificial Sequence

<220>
<223>  Biopolymer

<400>  24

Val Pro Gly Ile Gly Val Pro Gly Ile Gly Val Pro Gly Lys Gly Val
1               5                   10                  15

Pro Gly Ile Gly Val Pro Gly Ile Gly Val Pro Gly Ile Gly Val Pro
            20                  25                  30

Gly Ile Gly Val Pro Gly Lys Gly Val Pro Gly Ile Gly Val Pro Gly
        35                  40                  45

Ile Gly Ala Val Thr Gly Arg Gly Asp Ser Pro Ala Ser Ser Val Pro
    50                  55                  60

Gly Ile Gly Val Pro Gly Ile Gly Val Pro Gly Lys Gly Val Pro Gly
65                  70                  75                  80

Ile Gly Val Pro Gly Ile Gly Val Pro Gly Ile Gly Val Pro Gly Ile
                85                  90                  95

Gly Val Pro Gly Lys Gly Val Pro Gly Ile Gly Val Pro Gly Ile Gly
            100                 105                 110

Val Pro Gly Ile Gly Val Pro Gly Ile Gly Val Pro Gly Lys Gly Val
        115                 120                 125

Pro Gly Ile Gly Val Pro Gly Ile Gly Val Pro Gly Ile Gly Val Pro
    130                 135                 140

Gly Ile Gly Val Pro Gly Lys Gly Val Pro Gly Ile Gly Val Pro Gly
145                 150                 155                 160

Ile Gly Ala Val Thr Gly Arg Gly Asp Ser Pro Ala Ser Ser Val Pro
                165                 170                 175

Gly Ile Gly Val Pro Gly Ile Gly Val Pro Gly Lys Gly Val Pro Gly
            180                 185                 190

Ile Gly Val Pro Gly Ile Gly Val Pro Gly Ile Gly Val Pro Gly Ile
        195                 200                 205

Gly Val Pro Gly Lys Gly Val Pro Gly Ile Gly Val Pro Gly Ile Gly
    210                 215                 220

Val Pro Gly Ile Gly Val Pro Gly Ile Gly Val Pro Gly Lys Gly Val
225                 230                 235                 240

Pro Gly Ile Gly Val Pro Gly Ile Gly Val Pro Gly Ile Gly Val Pro
                245                 250                 255

Gly Ile Gly Val Pro Gly Lys Gly Val Pro Gly Ile Gly Val Pro Gly
            260                 265                 270

Ile Gly Ala Val Thr Gly Arg Gly Asp Ser Pro Ala Ser Ser Val Pro
        275                 280                 285

Gly Ile Gly Val Pro Gly Ile Gly Val Pro Gly Lys Gly Val Pro Gly
    290                 295                 300

Ile Gly Val Pro Gly Ile Gly Val Pro Gly Ile Gly Val Pro Gly Ile
305                 310                 315                 320

Gly Val Pro Gly Lys Gly Val Pro Gly Ile Gly Val Pro Gly Ile Gly
                325                 330                 335

Val Pro Gly Ile Gly Val Pro Gly Ile Gly Val Pro Gly Lys Gly Val
            340                 345                 350

Pro Gly Ile Gly Val Pro Gly Ile Gly Val Pro Gly Ile Gly Val Pro
        355                 360                 365

Gly Ile Gly Val Pro Gly Lys Gly Val Pro Gly Ile Gly Val Pro Gly
    370                 375                 380

Ile Gly Ala Val Thr Gly Arg Gly Asp Ser Pro Ala Ser Ser Val Pro
385                 390                 395                 400

Gly Ile Gly Val Pro Gly Ile Gly Val Pro Gly Lys Gly Val Pro Gly
                405                 410                 415

Ile Gly Val Pro Gly Ile Gly Val Pro Gly Ile Gly Val Pro Gly Ile
            420                 425                 430

Gly Val Pro Gly Lys Gly Val Pro Gly Ile Gly Val Pro Gly Ile Gly
        435                 440                 445

Val Pro Gly Ile Gly Val Pro Gly Ile Gly Val Pro Gly Lys Gly Val
    450                 455                 460

Pro Gly Ile Gly Val Pro Gly Ile Gly Val Pro Gly Ile Gly Val Pro
465                 470                 475                 480

Gly Ile Gly Val Pro Gly Lys Gly Val Pro Gly Ile Gly Val Pro Gly
                485                 490                 495

Ile Gly Ala Val Thr Gly Arg Gly Asp Ser Pro Ala Ser Ser Val Pro
            500                 505                 510

Gly Ile Gly Val Pro Gly Ile Gly Val Pro Gly Lys Gly Val Pro Gly
        515                 520                 525

Ile Gly Val Pro Gly Ile Gly Val Pro Gly Ile Gly Val Pro Gly Ile
    530                 535                 540

Gly Val Pro Gly Lys Gly Val Pro Gly Ile Gly Val Pro Gly Ile Gly
545                 550                 555                 560

Val Pro Gly Ile Gly Val Pro Gly Ile Gly Val Pro Gly Lys Gly Val
                565                 570                 575

Pro Gly Ile Gly Val Pro Gly Ile Gly Val Pro Gly Ile Gly Val Pro
            580                 585                 590

Gly Ile Gly Val Pro Gly Lys Gly Val Pro Gly Ile Gly Val Pro Gly
        595                 600                 605

Ile Gly Ala Val Thr Gly Arg Gly Asp Ser Pro Ala Ser Ser Val Pro
    610                 615                 620

Gly Ile Gly Val Pro Gly Ile Gly Val Pro Gly Lys Gly Val Pro Gly
625                 630                 635                 640

Ile Gly Val Pro Gly Ile Gly Val Pro Gly Ile Gly Val Pro Gly Ile
                645                 650                 655

Gly Val Pro Gly Lys Gly Val Pro Gly Ile Gly Val Pro Gly Ile Gly
            660                 665                 670

<210>  25
<211>  870
<212>  PRT
<213>  Artificial Sequence

<220>
<223>  Biopolymer

<400>  25

Val Pro Gly Ile Gly Val Pro Gly Ile Gly Val Pro Gly Lys Gly Val
1               5                   10                  15

Pro Gly Ile Gly Val Pro Gly Ile Gly Glu Glu Ile Gln Ile Gly His
            20                  25                  30

Ile Pro Arg Glu Asp Val Asp Tyr His Leu Tyr Pro Val Pro Gly Ile
        35                  40                  45

Gly Val Pro Gly Ile Gly Val Pro Gly Lys Gly Val Pro Gly Ile Gly
    50                  55                  60

Val Pro Gly Ile Gly Val Gly Val Ala Pro Gly Val Gly Val Ala Pro
65                  70                  75                  80

Gly Val Gly Val Ala Pro Gly Val Pro Gly Ile Gly Val Pro Gly Ile
                85                  90                  95

Gly Val Pro Gly Lys Gly Val Pro Gly Ile Gly Val Pro Gly Ile Gly
            100                 105                 110

Glu Glu Ile Gln Ile Gly His Ile Pro Arg Glu Asp Val Asp Tyr His
        115                 120                 125

Leu Tyr Pro Val Pro Gly Ile Gly Val Pro Gly Ile Gly Val Pro Gly
    130                 135                 140

Lys Gly Val Pro Gly Ile Gly Val Pro Gly Ile Gly Val Gly Val Ala
145                 150                 155                 160

Pro Gly Val Gly Val Ala Pro Gly Val Gly Val Ala Pro Gly Val Pro
                165                 170                 175

Gly Ile Gly Val Pro Gly Ile Gly Val Pro Gly Lys Gly Val Pro Gly
            180                 185                 190

Ile Gly Val Pro Gly Ile Gly Glu Glu Ile Gln Ile Gly His Ile Pro
        195                 200                 205

Arg Glu Asp Val Asp Tyr His Leu Tyr Pro Val Pro Gly Ile Gly Val
    210                 215                 220

Pro Gly Ile Gly Val Pro Gly Lys Gly Val Pro Gly Ile Gly Val Pro
225                 230                 235                 240

Gly Ile Gly Val Gly Val Ala Pro Gly Val Gly Val Ala Pro Gly Val
                245                 250                 255

Gly Val Ala Pro Gly Val Pro Gly Ile Gly Val Pro Gly Ile Gly Val
            260                 265                 270

Pro Gly Lys Gly Val Pro Gly Ile Gly Val Pro Gly Ile Gly Glu Glu
        275                 280                 285

Ile Gln Ile Gly His Ile Pro Arg Glu Asp Val Asp Tyr His Leu Tyr
    290                 295                 300

Pro Val Pro Gly Ile Gly Val Pro Gly Ile Gly Val Pro Gly Lys Gly
305                 310                 315                 320

Val Pro Gly Ile Gly Val Pro Gly Ile Gly Val Gly Val Ala Pro Gly
                325                 330                 335

Val Gly Val Ala Pro Gly Val Gly Val Ala Pro Gly Val Pro Gly Ile
            340                 345                 350

Gly Val Pro Gly Ile Gly Val Pro Gly Lys Gly Val Pro Gly Ile Gly
        355                 360                 365

Val Pro Gly Ile Gly Glu Glu Ile Gln Ile Gly His Ile Pro Arg Glu
    370                 375                 380

Asp Val Asp Tyr His Leu Tyr Pro Val Pro Gly Ile Gly Val Pro Gly
385                 390                 395                 400

Ile Gly Val Pro Gly Lys Gly Val Pro Gly Ile Gly Val Pro Gly Ile
                405                 410                 415

Gly Val Gly Val Ala Pro Gly Val Gly Val Ala Pro Gly Val Gly Val
            420                 425                 430

Ala Pro Gly Val Pro Gly Ile Gly Val Pro Gly Ile Gly Val Pro Gly
        435                 440                 445

Lys Gly Val Pro Gly Ile Gly Val Pro Gly Ile Gly Glu Glu Ile Gln
    450                 455                 460

Ile Gly His Ile Pro Arg Glu Asp Val Asp Tyr His Leu Tyr Pro Val
465                 470                 475                 480

Pro Gly Ile Gly Val Pro Gly Ile Gly Val Pro Gly Lys Gly Val Pro
                485                 490                 495

Gly Ile Gly Val Pro Gly Ile Gly Val Gly Val Ala Pro Gly Val Gly
            500                 505                 510

Val Ala Pro Gly Val Gly Val Ala Pro Gly Val Pro Gly Ile Gly Val
        515                 520                 525

Pro Gly Ile Gly Val Pro Gly Lys Gly Val Pro Gly Ile Gly Val Pro
    530                 535                 540

Gly Ile Gly Glu Glu Ile Gln Ile Gly His Ile Pro Arg Glu Asp Val
545                 550                 555                 560

Asp Tyr His Leu Tyr Pro Val Pro Gly Ile Gly Val Pro Gly Ile Gly
                565                 570                 575

Val Pro Gly Lys Gly Val Pro Gly Ile Gly Val Pro Gly Ile Gly Val
            580                 585                 590

Gly Val Ala Pro Gly Val Gly Val Ala Pro Gly Val Gly Val Ala Pro

595                 600                 605

Gly Val Pro Gly Ile Gly Val Pro Gly Ile Gly Val Pro Gly Lys Gly
    610                 615                 620

Val Pro Gly Ile Gly Val Pro Gly Ile Gly Glu Glu Ile Gln Ile Gly
625                 630                 635                 640

His Ile Pro Arg Glu Asp Val Asp Tyr His Leu Tyr Pro Val Pro Gly
                645                 650                 655

Ile Gly Val Pro Gly Ile Gly Val Pro Gly Lys Gly Val Pro Gly Ile
            660                 665                 670

Gly Val Pro Gly Ile Gly Val Gly Val Ala Pro Gly Val Gly Val Ala
        675                 680                 685

Pro Gly Val Gly Val Ala Pro Gly Val Pro Gly Ile Gly Val Pro Gly
    690                 695                 700

Ile Gly Val Pro Gly Lys Gly Val Pro Gly Ile Gly Val Pro Gly Ile
705                 710                 715                 720

Gly Glu Glu Ile Gln Ile Gly His Ile Pro Arg Glu Asp Val Asp Tyr
                725                 730                 735

His Leu Tyr Pro Val Pro Gly Ile Gly Val Pro Gly Ile Gly Val Pro
            740                 745                 750

Gly Lys Gly Val Pro Gly Ile Gly Val Pro Gly Ile Gly Val Gly Val
        755                 760                 765

Ala Pro Gly Val Gly Val Ala Pro Gly Val Gly Val Ala Pro Gly Val
    770                 775                 780

Pro Gly Ile Gly Val Pro Gly Ile Gly Val Pro Gly Lys Gly Val Pro
785                 790                 795                 800

Gly Ile Gly Val Pro Gly Ile Gly Glu Glu Ile Gln Ile Gly His Ile
                805                 810                 815

Pro Arg Glu Asp Val Asp Tyr His Leu Tyr Pro Val Pro Gly Ile Gly
            820                 825                 830

Val Pro Gly Ile Gly Val Pro Gly Lys Gly Val Pro Gly Ile Gly Val
        835                 840                 845

Pro Gly Ile Gly Val Gly Val Ala Pro Gly Val Gly Val Ala Pro Gly
    850                 855                 860

Val Gly Val Ala Pro Gly
865                 870

<210>  26
<211>  601
<212>  PRT
<213>  Artificial Sequence

<220>
<223>  Biopolymer

<400>  26

Val Pro Gly Ile Gly Val Pro Gly Ile Gly Val Pro Gly Lys Gly Val
1               5                   10                  15

Pro Gly Ile Gly Val Pro Gly Ile Gly Val Pro Gly Ile Gly Val Pro
            20                  25                  30

Gly Ile Gly Val Pro Gly Lys Gly Val Pro Gly Ile Gly Val Pro Gly
        35                  40                  45

Ile Gly Val Pro Gly Ile Gly Val Pro Gly Ile Gly Val Pro Gly Lys
    50                  55                  60

Gly Val Pro Gly Ile Gly Val Pro Gly Ile Gly Val Pro Gly Ile Gly
65                  70                  75                  80

Val Pro Gly Ile Gly Val Pro Gly Lys Gly Val Pro Gly Ile Gly Val
                85                  90                  95

Pro Gly Ile Gly Val Pro Gly Ile Gly Val Pro Gly Ile Gly Val Pro
            100                 105                 110

Gly Lys Gly Val Pro Gly Ile Gly Val Pro Gly Ile Gly Val Pro Gly
        115                 120                 125

Ile Gly Val Pro Gly Ile Gly Val Pro Gly Lys Gly Val Pro Gly Ile
    130                 135                 140

Gly Val Pro Gly Ile Gly Val Pro Gly Ile Gly Val Pro Gly Ile Gly
145                 150                 155                 160

Val Pro Gly Lys Gly Val Pro Gly Ile Gly Val Pro Gly Ile Gly Val
                165                 170                 175

Pro Gly Ile Gly Val Pro Gly Ile Gly Val Pro Gly Lys Gly Val Pro

180                 185                 190

Gly Ile Gly Val Pro Gly Ile Gly Val Pro Gly Ile Gly Val Pro Gly
        195                 200                 205

Ile Gly Val Pro Gly Lys Gly Val Pro Gly Ile Gly Val Pro Gly Ile
    210                 215                 220

Gly Val Pro Gly Ile Gly Val Pro Gly Ile Gly Val Pro Gly Lys Gly
225                 230                 235                 240

Val Pro Gly Ile Gly Val Pro Gly Ile Gly Val Pro Gly Ile Gly Val
                245                 250                 255

Pro Gly Ile Gly Val Pro Gly Lys Gly Val Pro Gly Ile Gly Val Pro
            260                 265                 270

Gly Ile Gly Val Pro Gly Ile Gly Val Pro Gly Ile Gly Val Pro Gly
        275                 280                 285

Lys Gly Val Pro Gly Ile Gly Val Pro Gly Ile Gly Val Pro Gly Ile
    290                 295                 300

Gly Val Pro Gly Ile Gly Val Pro Gly Lys Gly Val Pro Gly Ile Gly
305                 310                 315                 320

Val Pro Gly Ile Gly Val Pro Gly Ile Gly Val Pro Gly Ile Gly Val
                325                 330                 335

Pro Gly Lys Gly Val Pro Gly Ile Gly Val Pro Gly Ile Gly Val Pro
            340                 345                 350

Gly Ile Gly Val Pro Gly Ile Gly Val Pro Gly Lys Gly Val Pro Gly
        355                 360                 365

Ile Gly Val Pro Gly Ile Gly Val Pro Gly Ile Gly Val Pro Gly Ile
    370                 375                 380

Gly Val Pro Gly Lys Gly Val Pro Gly Ile Gly Val Pro Gly Ile Gly
385                 390                 395                 400

Val Pro Gly Ile Gly Val Pro Gly Ile Gly Val Pro Gly Lys Gly Val
                405                 410                 415

Pro Gly Ile Gly Val Pro Gly Ile Gly Val Pro Gly Ile Gly Val Pro
            420                 425                 430

Gly Ile Gly Val Pro Gly Lys Gly Val Pro Gly Ile Gly Val Pro Gly
        435                 440                 445

Ile Gly Val Pro Gly Ile Gly Val Pro Gly Ile Gly Val Pro Gly Lys
    450                 455                 460

Gly Val Pro Gly Ile Gly Val Pro Gly Ile Gly Val Pro Gly Ile Gly
465                 470                 475                 480

Val Pro Gly Ile Gly Val Pro Gly Lys Gly Val Pro Gly Ile Gly Val
                485                 490                 495

Pro Gly Ile Gly Val Pro Gly Ile Gly Val Pro Gly Ile Gly Val Pro
            500                 505                 510

Gly Lys Gly Val Pro Gly Ile Gly Val Pro Gly Ile Gly Val Pro Gly
        515                 520                 525

Ile Gly Val Pro Gly Ile Gly Val Pro Gly Lys Gly Val Pro Gly Ile
    530                 535                 540

Gly Val Pro Gly Ile Gly Val Pro Gly Ile Gly Val Pro Gly Ile Gly
545                 550                 555                 560

Val Pro Gly Lys Gly Val Pro Gly Ile Gly Val Pro Gly Ile Gly Val
                565                 570                 575

Pro Gly Ile Gly Val Pro Gly Ile Gly Val Pro Gly Lys Gly Val Pro
            580                 585                 590

Gly Ile Gly Val Pro Gly Ile Gly Val
        595                 600

<210>  27
<211>  6
<212>  PRT
<213>  Artificial Sequence

<220>
<223>  Elastin-like protein domain

<400>  27

Ala Pro Gly Val Gly Val
1               5

<210>  28
<211>  2040
<212>  DNA
<213>  Artificial Sequence

<220>
<223>  Biopolymer

<400>  28
gtaccgggta ttggcgtgcc gggtatcggc gttccaggta aaggggtgcc gggcattggt      60

gtcccgggca tcggtgtgcc gggcattggt gttcctggca taggtgtacc ggggaaaggt     120

gtgccaggta ttggcgtgcc tgggatcggt gtgggtgtcg caccgggggt aggcgttgcc     180

ccgggcgtcg gtgttgcgcc aggcgtaccg ggtattggcg tgccgggtat cggcgttcca     240

ggtaaagggg tgccgggcat tggtgtcccg ggcatcggtg tgccgggcat tggtgttcct     300

ggcataggtg taccggggaa aggtgtgcca ggtattggcg tgcctgggat cggtgtgggt     360

gtcgcaccgg gggtaggcgt tgccccgggc gtcggtgttg cgccaggcgt accgggtatt     420

ggcgtgccgg gtatcggcgt tccaggtaaa ggggtgccgg gcattggtgt cccgggcatc     480

ggtgtgccgg gcattggtgt tcctggcata ggtgtaccgg ggaaaggtgt gccaggtatt     540

ggcgtgcctg ggatcggtgt gggtgtcgca ccgggggtag gcgttgcccc gggcgtcggt     600

gttgcgccag gcgtaccggg tattggcgtg ccgggtatcg gcgttccagg taaaggggtg     660

ccgggcattg gtgtcccggg catcggtgtg ccgggcattg gtgttcctgg cataggtgta     720

ccggggaaag gtgtgccagg tattggcgtg cctgggatcg gtgtgggtgt cgcaccgggg     780

gtaggcgttg ccccgggcgt cggtgttgcg ccaggcgtac cgggtattgg cgtgccgggt     840

atcggcgttc caggtaaagg ggtgccgggc attggtgtcc cgggcatcgg tgtgccgggc     900

attggtgttc ctggcatagg tgtaccgggg aaaggtgtgc caggtattgg cgtgcctggg     960

atcggtgtgg gtgtcgcacc gggggtaggc gttgccccgg gcgtcggtgt tgcgccaggc    1020

gtaccgggta ttggcgtgcc gggtatcggc gttccaggta aaggggtgcc gggcattggt    1080

gtcccgggca tcggtgtgcc gggcattggt gttcctggca taggtgtacc ggggaaaggt    1140

gtgccaggta ttggcgtgcc tgggatcggt gtgggtgtcg caccgggggt aggcgttgcc    1200

ccgggcgtcg gtgttgcgcc aggcgtaccg ggtattggcg tgccgggtat cggcgttcca    1260

ggtaaagggg tgccgggcat tggtgtcccg ggcatcggtg tgccgggcat tggtgttcct    1320

ggcataggtg taccggggaa aggtgtgcca ggtattggcg tgcctgggat cggtgtgggt    1380

gtcgcaccgg gggtaggcgt tgccccgggc gtcggtgttg cgccaggcgt accgggtatt    1440

ggcgtgccgg gtatcggcgt tccaggtaaa ggggtgccgg gcattggtgt cccgggcatc    1500

ggtgtgccgg gcattggtgt tcctggcata ggtgtaccgg ggaaaggtgt gccaggtatt    1560

ggcgtgcctg ggatcggtgt gggtgtcgca ccgggggtag gcgttgcccc gggcgtcggt    1620

gttgcgccag gcgtaccggg tattggcgtg ccgggtatcg gcgttccagg taaaggggtg    1680

ccgggcattg gtgtcccggg catcggtgtg ccgggcattg gtgttcctgg cataggtgta    1740

ccggggaaag gtgtgccagg tattggcgtg cctgggatcg gtgtgggtgt cgcaccgggg    1800

gtaggcgttg ccccgggcgt cggtgttgcg ccaggcgtac cgggtattgg cgtgccgggt 1860

atcggcgttc caggtaaagg ggtgccgggc attggtgtcc cgggcatcgg tgtgccgggc 1920

attggtgttc ctggcatagg tgtaccgggg aaaggtgtgc caggtattgg cgtgcctggg 1980

atcggtgtgg gtgtcgcacc gggggtaggc gttgccccgg gcgtcggtgt tgcgccaggc 2040

<210> 29
<211> 1416
<212> DNA
<213> Artificial Sequence

<220>
<223> Biopolymer

<400> 29
gtaccgggca ttggtgttcc gggcatcggt gtgccgggca aaggtgttcc gggcattggt 60

gtgccgggca tcggtgtgcc aggcattggt gtgccgggca tcggtgttcc gggcaagggt 120

gtgccgggca tcggtgtgcc aggcattggt gtatacgcag ttaccggtga ccgtatccgt 180

tccgcttctc cggcgtcctc tgcagtaccg ggcattggtg ttccgggcat cggtgtgccg 240

ggcaaaggtg ttccgggcat tggtgtgccg ggcatcggtg tgccaggcat tggtgtgccg 300

ggcatcggtg ttccgggcaa gggtgtgccg ggcatcggtg tgccaggcat tggtgtaccg 360

ggcattggtg ttccgggcat cggtgtgccg ggcaaaggtg ttccgggcat tggtgtgccg 420

ggcatcggtg tgccaggcat tggtgtgccg ggcatcggtg ttccgggcaa gggtgtgccg 480

ggcatcggtg tgccaggcat tggtgtatac gcagttaccg gtgaccgtat ccgttccgct 540

tctccggcgt cctctgcagt accgggcatt ggtgttccgg gcatcggtgt gccgggcaaa 600

ggtgttccgg gcattggtgt gccgggcatc ggtgtgccag gcattggtgt gccgggcatc 660

ggtgttccgg gcaagggtgt gccgggcatc ggtgtgccag gcattggtgt accgggcatt 720

ggtgttccgg gcatcggtgt gccgggcaaa ggtgttccgg gcattggtgt gccgggcatc 780

ggtgtgccag gcattggtgt gccgggcatc ggtgttccgg gcaagggtgt gccgggcatc 840

ggtgtgccag gcattggtgt atacgcagtt accggtgacc gtatccgttc cgcttctccg 900

gcgtcctctg cagtaccggg cattggtgtt ccgggcatcg gtgtgccggg caaaggtgtt 960

ccgggcattg gtgtgccggg catcggtgtg ccaggcattg gtgtgccggg catcggtgtt 1020

ccgggcaagg gtgtgccggg catcggtgtg ccaggcattg gtgtaccggg cattggtgtt 1080

ccgggcatcg gtgtgccggg caaaggtgtt ccgggcattg gtgtgccggg catcggtgtg 1140

ccaggcattg gtgtgccggg catcggtgtt ccgggcaagg gtgtgccggg catcggtgtg 1200

ccaggcattg gtgtatacgc agttaccggt gaccgtatcc gttccgcttc tccggcgtcc 1260

tctgcagtac cgggcattgg tgttccgggc atcggtgtgc cgggcaaagg tgttccgggc 1320

attggtgtgc cgggcatcgg tgtgccaggc attggtgtgc cgggcatcgg tgttccgggc 1380

aagggtgtgc cgggcatcgg tgtgccaggc attggt 1416

<210> 30
<211> 1416
<212> DNA
<213> Artificial Sequence

<220>
<223> Biopolymer

<400> 30
gtaccgggca ttggtgttcc gggcatcggt gtgccgggca aaggtgttcc gggcattggt 60

gtgccgggca tcggtgtgcc aggcattggt gtgccgggca tcggtgttcc gggcaagggt 120

gtgccgggca tcggtgtgcc aggcattggt gtatacgcag ttaccggtga ccgtatccgt 180

tccgcttctc cggcgtcctc tgcagtaccg ggcattggtg ttccgggcat cggtgtgccg 240

ggcaaaggtg ttccgggcat tggtgtgccg ggcatcggtg tgccaggcat tggtgtgccg 300

ggcatcggtg ttccgggcaa gggtgtgccg ggcatcggtg tgccaggcat tggtgtaccg 360

ggcattggtg ttccgggcat cggtgtgccg ggcaaaggtg ttccgggcat tggtgtgccg 420

ggcatcggtg tgccaggcat tggtgtgccg ggcatcggtg ttccgggcaa gggtgtgccg 480

ggcatcggtg tgccaggcat tggtgtatac gcagttaccg gtgaccgtat ccgttccgct 540

tctccggcgt cctctgcagt accgggcatt ggtgttccgg gcatcggtgt gccgggcaaa 600

ggtgttccgg gcattggtgt gccgggcatc ggtgtgccag gcattggtgt gccgggcatc 660

ggtgttccgg gcaagggtgt gccgggcatc ggtgtgccag gcattggtgt accgggcatt 720

ggtgttccgg gcatcggtgt gccgggcaaa ggtgttccgg gcattggtgt gccgggcatc 780

ggtgtgccag gcattggtgt gccgggcatc ggtgttccgg gcaagggtgt gccgggcatc 840

ggtgtgccag gcattggtgt atacgcagtt accggtgacc gtatccgttc cgcttctccg 900

gcgtcctctg cagtaccggg cattggtgtt ccgggcatcg gtgtgccggg caaaggtgtt 960

ccgggcattg gtgtgccggg catcggtgtg ccaggcattg gtgtgccggg catcggtgtt 1020

ccgggcaagg gtgtgccggg catcggtgtg ccaggcattg gtgtaccggg cattggtgtt 1080

ccgggcatcg gtgtgccggg caaaggtgtt ccgggcattg gtgtgccggg catcggtgtg 1140

ccaggcattg gtgtgccggg catcggtgtt ccgggcaagg gtgtgccggg catcggtgtg 1200

ccaggcattg gtgtatacgc agttaccggt gaccgtatcc gttccgcttc tccggcgtcc 1260

tctgcagtac cgggcattgg tgttccgggc atcggtgtgc cgggcaaagg tgttccgggc 1320

attggtgtgc cgggcatcgg tgtgccaggc attggtgtgc cgggcatcgg tgttccgggc 1380

aagggtgtgc cgggcatcgg tgtgccaggc attggt 1416

<210>  31
<211>  1416
<212>  DNA
<213>  Artificial Sequence

<220>
<223>  Biopolymer

<400>  31
gtaccgggca ttggtgttcc gggcatcggt gtgccgggca aaggtgttcc gggcattggt      60

gtgccgggca tcggtgtgcc aggcattggt gtgccgggca tcggtgttcc gggcaagggt     120

gtgccgggca tcggtgtgcc aggcattggt gtatacgcag ttaccggtga caaccgtcgt     180

tccgcttctc cggcgtcctc tgcagtaccg ggcattggtg ttccgggcat cggtgtgccg     240

ggcaaaggtg ttccgggcat tggtgtgccg ggcatcggtg tgccaggcat tggtgtgccg     300

ggcatcggtg ttccgggcaa gggtgtgccg ggcatcggtg tgccaggcat tggtgtaccg     360

ggcattggtg ttccgggcat cggtgtgccg ggcaaaggtg ttccgggcat tggtgtgccg     420

ggcatcggtg tgccaggcat tggtgtgccg ggcatcggtg ttccgggcaa gggtgtgccg     480

ggcatcggtg tgccaggcat tggtgtatac gcagttaccg gtgacaaccg tcgttccgct     540

tctccggcgt cctctgcagt accgggcatt ggtgttccgg gcatcggtgt gccgggcaaa     600

ggtgttccgg gcattggtgt gccgggcatc ggtgtgccag gcattggtgt gccgggcatc     660

ggtgttccgg gcaagggtgt gccgggcatc ggtgtgccag gcattggtgt accgggcatt     720

ggtgttccgg gcatcggtgt gccgggcaaa ggtgttccgg gcattggtgt gccgggcatc     780

ggtgtgccag gcattggtgt gccgggcatc ggtgttccgg gcaagggtgt gccgggcatc     840

ggtgtgccag gcattggtgt atacgcagtt accggtgaca accgtcgttc cgcttctccg     900

gcgtcctctg cagtaccggg cattggtgtt ccgggcatcg gtgtgccggg caaaggtgtt     960

ccgggcattg gtgtgccggg catcggtgtg ccaggcattg gtgtgccggg catcggtgtt    1020

ccgggcaagg gtgtgccggg catcggtgtg ccaggcattg gtgtaccggg cattggtgtt    1080

ccgggcatcg gtgtgccggg caaaggtgtt ccgggcattg gtgtgccggg catcggtgtg    1140

ccaggcattg gtgtgccggg catcggtgtt ccgggcaagg gtgtgccggg catcggtgtg    1200

ccaggcattg gtgtatacgc agttaccggt gacaaccgtc gttccgcttc tccggcgtcc    1260

tctgcagtac cgggcattgg tgttccgggc atcggtgtgc cgggcaaagg tgttccgggc    1320

attggtgtgc cgggcatcgg tgtgccaggc attggtgtgc cgggcatcgg tgttccgggc    1380

aagggtgtgc cgggcatcgg tgtgccaggc attggt                              1416

<210>  32
<211>  1416
<212>  DNA
<213>  Artificial Sequence

<220>
<223>  Biopolymer

<400>  32
gtaccgggca ttggtgttcc gggcatcggt gtgccgggca aaggtgttcc gggcattggt      60

gtgccgggca tcggtgtgcc aggcattggt gtgccgggca tcggtgttcc gggcaagggt     120

gtgccgggca tcggtgtgcc aggcattggt gtatacgcag ttaccggttt tttctcccgc     180

tccgcttctc cggcgtcctc tgcagtaccg ggcattggtg ttccgggcat cggtgtgccg     240

ggcaaaggtg ttccgggcat tggtgtgccg ggcatcggtg tgccaggcat tggtgtgccg     300

ggcatcggtg ttccgggcaa gggtgtgccg ggcatcggtg tgccaggcat tggtgtaccg     360

ggcattggtg ttccgggcat cggtgtgccg ggcaaaggtg ttccgggcat tggtgtgccg     420

ggcatcggtg tgccaggcat tggtgtgccg ggcatcggtg ttccgggcaa gggtgtgccg     480

ggcatcggtg tgccaggcat tggtgtatac gcagttaccg gttttttctc ccgctccgct     540

tctccggcgt cctctgcagt accgggcatt ggtgttccgg gcatcggtgt gccgggcaaa     600

ggtgttccgg gcattggtgt gccgggcatc ggtgtgccag gcattggtgt gccgggcatc     660

ggtgttccgg gcaagggtgt gccgggcatc ggtgtgccag gcattggtgt accgggcatt     720

ggtgttccgg gcatcggtgt gccgggcaaa ggtgttccgg gcattggtgt gccgggcatc     780

ggtgtgccag gcattggtgt gccgggcatc ggtgttccgg gcaagggtgt gccgggcatc     840

ggtgtgccag gcattggtgt atacgcagtt accggttttt tctcccgctc cgcttctccg     900

gcgtcctctg cagtaccggg cattggtgtt ccgggcatcg gtgtgccggg caaaggtgtt     960

ccgggcattg gtgtgccggg catcggtgtg ccaggcattg gtgtgccggg catcggtgtt    1020

ccgggcaagg gtgtgccggg catcggtgtg ccaggcattg gtgtaccggg cattggtgtt    1080

ccgggcatcg gtgtgccggg caaaggtgtt ccgggcattg gtgtgccggg catcggtgtg    1140

ccaggcattg gtgtgccggg catcggtgtt ccgggcaagg gtgtgccggg catcggtgtg    1200

ccaggcattg gtgtatacgc agttaccggt tttttctccc gctccgcttc tccggcgtcc    1260

tctgcagtac cgggcattgg tgttccgggc atcggtgtgc cgggcaaagg tgttccgggc    1320

attggtgtgc cgggcatcgg tgtgccaggc attggtgtgc cgggcatcgg tgttccgggc    1380

aagggtgtgc cgggcatcgg tgtgccaggc attggt                              1416


<210>  33
<211>  1962
<212>  DNA
<213>  Artificial Sequence

<220>
<223>  Biopolymer

<400>  33
atgggcagca gccatcatca tcatcatcac caccacggcc tggtgccgcg cggcagccat      60

atgggtaaaa aaaagccggt accaggtgtt ggtgttccgg gtgttggcgt gccgggcgaa 120

ggcgtgccgg gtgttggtgt tccgggtgta ggggtaccag gtgttggtgt tccgggtgtt 180

ggcgtgccgg gcgaaggcgt gccgggtgtt ggtgttccgg gtgtaggggt accaggtgtt 240

ggtgttccgg gtgttggcgt gccgggcgaa ggcgtgccgg gtgttggtgt tccgggtgta 300

ggggtaccag gtgttggtgt tccgggtgtt ggcgtgccgg gcgaaggcgt gccgggtgtt 360

ggtgttccgg gtgtaggggt accaggtgtt ggtgttccgg gtgttggcgt gccgggcgaa 420

ggcgtgccgg gtgttggtgt tccgggtgta ggggtagtac caggtgttgg tgttccgggt 480

gttggcgtgc cgggcgaagg cgtgccgggt gttggtgttc cgggtgtagg ggtaccaggt 540

gttggtgttc cgggtgttgg cgtgccgggc gaaggcgtgc cgggtgttgg tgttccgggt 600

gtaggggtac caggtgttgg tgttccgggt gttggcgtgc cgggcgaagg cgtgccgggt 660

gttggtgttc cgggtgtagg ggtaccaggt gttggtgttc cgggtgttgg cgtgccgggc 720

gaaggcgtgc cgggtgttgg tgttccgggt gtaggggtac caggtgttgg tgttccgggt 780

gttggcgtgc cgggcgaagg cgtgccgggt gttggtgttc cgggtgtagg ggtaggcggt 840

ggtggtggcc cgatgggccc gtctggtccc tggggtggtg gcggtggtgt aggcggtggt 900

ggccagccgc agggtctggc taaaggtggt ggtggtggtg taggcggtgg tggtggcccg 960

cagggcatct ggggtcaggg tggcggtggt gtaccaggtg ttggtgttcc gggtgttggc 1020

gtgccgggcg aaggcgtgcc gggtgttggt gttccgggtg taggggtacc aggtgttggt 1080

gttccgggtg ttggcgtgcc gggcgaaggc gtgccgggtg ttggtgttcc gggtgtaggg 1140

gtaccaggtg ttggtgttcc gggtgttggc gtgccgggcg aaggcgtgcc gggtgttggt 1200

gttccgggtg taggggtacc aggtgttggt gttccgggtg ttggcgtgcc gggcgaaggc 1260

gtgccgggtg ttggtgttcc gggtgtaggg gtaccaggtg ttggtgttcc gggtgttggc 1320

gtgccgggcg aaggcgtgcc gggtgttggt gttccgggtg taggggtagt accaggtgtt 1380

ggtgttccgg gtgttggcgt gccgggcgaa ggcgtgccgg gtgttggtgt tccgggtgta 1440

ggggtaccag gtgttggtgt tccgggtgtt ggcgtgccgg gcgaaggcgt gccgggtgtt 1500

ggtgttccgg gtgtaggggt accaggtgtt ggtgttccgg gtgttggcgt gccgggcgaa 1560

ggcgtgccgg gtgttggtgt tccgggtgta ggggtaccag gtgttggtgt tccgggtgtt 1620

ggcgtgccgg gcgaaggcgt gccgggtgtt ggtgttccgg gtgtaggggt accaggtgtt 1680

ggtgttccgg gtgttggcgt gccgggcgaa ggcgtgccgg gtgttggtgt tccgggtgta 1740

ggggtaggcg gtggtggtgg cccgatgggc ccgtctggtc cctggggtgg tggcggtggt 1800

gtaggcggtg gtggccagcc gcagggtctg gctaaaggtg gtggtggtgg tgtaggcggt 1860

ggtggtggcc cgcagggcat ctggggtcag ggtggcggtg gtgtaggcgg tggtggtggc 1920

aaaaaaaaag gtggtggcgg tggtgtagta aaaaagaaat ga 1962

<210> 34
<211> 2016
<212> DNA
<213> Artificial Sequence

<220>
<223> Biopolymer

<400> 34
gtaccgggca tcggtgttcc gggcattggt gtgccgggca aaggtgttcc gggcattggt 60

gtgccgggca tcggtgtgcc aggcattggt gtgccgggca tcggtgttcc gggcaagggt 120

gtgccgggca ttggtgtgcc aggcatcggt gcagtaaccg gtcgtgggga ttctcctgcg 180

tccagcgtcc cgggcatcgg tgttccgggc attggtgtgc cgggcaaagg tgttccgggc 240

attggtgtgc cgggcatcgg tgtgccaggc attggtgtgc cgggcatcgg tgttccgggc 300

aaaggtgtgc caggcatcgg tgtgccgggc attggtgtac cgggcatcgg tgttccgggc 360

attggtgtgc cgggcaaagg tgttccgggc attggtgtgc cgggcatcgg tgtgccaggc 420

attggtgtgc cgggcatcgg tgttccgggc aagggtgtgc cgggcattgg tgtgccaggc 480

atcggtgcag taaccggtcg tggggattct cctgcgtcca gcgtcccggg catcggtgtt 540

ccgggcattg gtgtgccggg caaaggtgtt ccgggcattg gtgtgccggg catcggtgtg 600

ccaggcattg gtgtgccggg catcggtgtt ccgggcaaag gtgtgccagg catcggtgtg 660

ccgggcattg gtgtaccggg catcggtgtt ccgggcattg gtgtgccggg caaaggtgtt 720

ccgggcattg gtgtgccggg catcggtgtg ccaggcattg gtgtgccggg catcggtgtt 780

ccgggcaagg gtgtgccggg cattggtgtg ccaggcatcg gtgcagtaac cggtcgtggg 840

gattctcctg cgtccagcgt cccgggcatc ggtgttccgg gcattggtgt gccgggcaaa 900

ggtgttccgg gcattggtgt gccgggcatc ggtgtgccag gcattggtgt gccgggcatc 960

ggtgttccgg gcaaaggtgt gccaggcatc ggtgtgccgg gcattggtgt accgggcatc 1020

ggtgttccgg gcattggtgt gccgggcaaa ggtgttccgg gcattggtgt gccgggcatc 1080

ggtgtgccag gcattggtgt gccgggcatc ggtgttccgg gcaagggtgt gccgggcatt 1140

ggtgtgccag gcatcggtgc agtaaccggt cgtggggatt ctcctgcgtc cagcgtcccg 1200

ggcatcggtg ttccgggcat tggtgtgccg ggcaaaggtg ttccgggcat tggtgtgccg 1260

ggcatcggtg tgccaggcat tggtgtgccg ggcatcggtg ttccgggcaa aggtgtgcca 1320

ggcatcggtg tgccgggcat tggtgtaccg ggcatcggtg ttccgggcat tggtgtgccg 1380

ggcaaaggtg ttccgggcat tggtgtgccg ggcatcggtg tgccaggcat tggtgtgccg 1440

ggcatcggtg ttccgggcaa gggtgtgccg ggcattggtg tgccaggcat cggtgcagta 1500

accggtcgtg gggattctcc tgcgtccagc gtcccgggca tcggtgttcc gggcattggt 1560

gtgccgggca aaggtgttcc gggcattggt gtgccgggca tcggtgtgcc aggcattggt 1620

gtgccgggca tcggtgttcc gggcaaaggt gtgccaggca tcggtgtgcc gggcattggt 1680

gtaccgggca tcggtgttcc gggcattggt gtgccgggca aaggtgttcc gggcattggt 1740

gtgccgggca tcggtgtgcc aggcattggt gtgccgggca tcggtgttcc gggcaagggt 1800

gtgccgggca ttggtgtgcc aggcatcggt gcagtaaccg gtcgtgggga ttctcctgcg 1860

tccagcgtcc cgggcatcgg tgttccgggc attggtgtgc cgggcaaagg tgttccgggc 1920

attggtgtgc cgggcatcgg tgtgccaggc attggtgtgc cgggcatcgg tgttccgggc 1980

aaaggtgtgc caggcatcgg tgtgccgggc attggt 2016

<210> 35
<211> 2607
<212> DNA
<213> Artificial Sequence

<220>
<223> Biopolymer

<400> 35
ccgggcatcg gtgttccggg cattggtgtg ccgggcaaag gtgttccggg cattggtgtg 60

ccgggcattg gtgaagaaat ccagatcggg catatcccac gcgaggatgt ggactaccac 120

ctgtatccgg tgccaggcat tggtgtgccg ggcattggtg ttccgggcaa aggtgtgccg 180

ggcattggtg ttccgggcat cggtgtgggc gttgcgccgg gtgtgggcgt tgccccgggt 240

gtgggcgttg caccgggtgt accgggcatc ggtgttccgg gcattggtgt gccgggcaaa 300

ggtgttccgg gcattggtgt gccgggcatt ggtgaagaaa tccagatcgg gcatatccca 360

cgcgaggatg tggactacca cctgtatccg gtgccaggca ttggtgtgcc gggcattggt 420

gttccgggca aaggtgtgcc gggcattggt gttccgggca tcggtgtggg cgttgcgccg 480

ggtgtgggcg ttgccccggg tgtgggcgtt gcaccgggtg taccgggcat cggtgttccg 540

ggcattggtg tgccgggcaa aggtgttccg ggcattggtg tgccgggcat tggtgaagaa 600

atccagatcg ggcatatccc acgcgaggat gtggactacc acctgtatcc ggtgccaggc 660

attggtgtgc cgggcattgg tgttccgggc aaaggtgtgc cgggcattgg tgttccgggc 720

atcggtgtgg gcgttgcgcc gggtgtgggc gttgccccgg gtgtgggcgt tgcaccgggt 780

gtaccgggca tcggtgttcc gggcattggt gtgccgggca aaggtgttcc gggcattggt 840

gtgccgggca ttggtgaaga aatccagatc gggcatatcc cacgcgagga tgtggactac 900

cacctgtatc cggtgccagg cattggtgtg ccgggcattg gtgttccggg caaaggtgtg 960

ccgggcattg gtgttccggg catcggtgtg ggcgttgcgc cgggtgtggg cgttgccccg 1020

ggtgtgggcg ttgcaccggg tgtaccgggc atcggtgttc cgggcattgg tgtgccgggc 1080

aaaggtgttc cgggcattgg tgtgccgggc attggtgaag aaatccagat cgggcatatc 1140

ccacgcgagg atgtggacta ccacctgtat ccggtgccag gcattggtgt gccgggcatt 1200

ggtgttccgg gcaaaggtgt gccgggcatt ggtgttccgg gcatcggtgt gggcgttgcg 1260

ccgggtgtgg gcgttgcccc gggtgtgggc gttgcaccgg gtgtaccggg catcggtgtt 1320

ccgggcattg gtgtgccggg caaaggtgtt ccgggcattg gtgtgccggg cattggtgaa 1380

gaaatccaga tcgggcatat cccacgcgag gatgtggact accacctgta tccggtgcca 1440

ggcattggtg tgccgggcat tggtgttccg ggcaaaggtg tgccgggcat tggtgttccg 1500

ggcatcggtg tgggcgttgc gccgggtgtg ggcgttgccc cgggtgtggg cgttgcaccg 1560

ggtgtaccgg gcatcggtgt tccgggcatt ggtgtgccgg gcaaaggtgt tccgggcatt 1620

ggtgtgccgg gcattggtga agaaatccag atcgggcata tcccacgcga ggatgtggac 1680

taccacctgt atccggtgcc aggcattggt gtgccgggca ttggtgttcc gggcaaaggt 1740

gtgccgggca ttggtgttcc gggcatcggt gtgggcgttg cgccgggtgt gggcgttgcc 1800

ccgggtgtgg gcgttgcacc gggtgtaccg ggcatcggtg ttccgggcat tggtgtgccg 1860

ggcaaaggtg ttccgggcat tggtgtgccg ggcattggtg aagaaatcca gatcgggcat 1920

atcccacgcg aggatgtgga ctaccacctg tatccggtgc caggcattgg tgtgccgggc 1980

attggtgttc cgggcaaagg tgtgccgggc attggtgttc cgggcatcgg tgtgggcgtt 2040

gcgccgggtg tgggcgttgc cccgggtgtg ggcgttgcac cgggtgtacc gggcatcggt 2100

gttccgggca ttggtgtgcc gggcaaaggt gttccgggca ttggtgtgcc gggcattggt 2160

gaagaaatcc agatcgggca tatcccacgc gaggatgtgg actaccacct gtatccggtg 2220

ccaggcattg gtgtgccggg cattggtgtt ccgggcaaag gtgtgccggg cattggtgtt 2280

ccgggcatcg gtgtgggcgt tgcgccgggt gtgggcgttg ccccgggtgt gggcgttgca 2340

ccgggtgtac cgggcatcgg tgttccgggc attggtgtgc cgggcaaagg tgttccgggc 2400

attggtgtgc cgggcattgg tgaagaaatc cagatcgggc atatcccacg cgaggatgtg 2460

gactaccacc tgtatccggt gccaggcatt ggtgtgccgg gcattggtgt tccgggcaaa 2520

ggtgtgccgg gcattggtgt tccgggcatc ggtgtgggcg ttgcgccggg tgtgggcgtt 2580

gccccgggtg tgggcgttgc accgggt 2607

<210> 36
<211> 3600
<212> DNA
<213> Artificial Sequence

<220>
<223> Biopolymer

<400> 36
gtaccgggca ttggtgttcc gggcatcggt gtgccgggca aaggtgttcc gggcattggt 60

gtgccgggca tcggtgtgcc aggcattggt gtgccgggca tcggtgttcc gggcaagggt 120

gtgccgggca tcggtgtgcc aggcattggt gtaccgggca ttggtgttcc gggcatcggt 180

gtgccgggca aaggtgttcc gggcattggt gtgccgggca tcggtgtgcc aggcattggt 240

gtgccgggca tcggtgttcc gggcaagggt gtgccgggca tcggtgtgcc aggcattggt 300

gtaccgggca ttggtgttcc gggcatcggt gtgccgggca aaggtgttcc gggcattggt 360

gtgccgggca tcggtgtgcc aggcattggt gtgccgggca tcggtgttcc gggcaagggt 420

gtgccgggca tcggtgtgcc aggcattggt gtaccgggca ttggtgttcc gggcatcggt 480

gtgccgggca aaggtgttcc gggcattggt gtgccgggca tcggtgtgcc aggcattggt 540

gtgccgggca tcggtgttcc gggcaagggt gtgccgggca tcggtgtgcc aggcattggt 600

gtaccgggca ttggtgttcc gggcatcggt gtgccgggca aaggtgttcc gggcattggt 660

gtgccgggca tcggtgtgcc aggcattggt gtgccgggca tcggtgttcc gggcaagggt 720

gtgccgggca tcggtgtgcc aggcattggt gtaccgggca ttggtgttcc gggcatcggt 780

gtgccgggca aaggtgttcc gggcattggt gtgccgggca tcggtgtgcc aggcattggt 840

gtgccgggca tcggtgttcc gggcaagggt gtgccgggca tcggtgtgcc aggcattggt 900

gtaccgggca ttggtgttcc gggcatcggt gtgccgggca aaggtgttcc gggcattggt 960

gtgccgggca tcggtgtgcc aggcattggt gtgccgggca tcggtgttcc gggcaagggt 1020

gtgccgggca tcggtgtgcc aggcattggt gtaccgggca ttggtgttcc gggcatcggt 1080

gtgccgggca aaggtgttcc gggcattggt gtgccgggca tcggtgtgcc aggcattggt 1140

gtgccgggca tcggtgttcc gggcaagggt gtgccgggca tcggtgtgcc aggcattggt 1200

gtaccgggca ttggtgttcc gggcatcggt gtgccgggca aaggtgttcc gggcattggt 1260

gtgccgggca tcggtgtgcc aggcattggt gtgccgggca tcggtgttcc gggcaagggt 1320

gtgccgggca tcggtgtgcc aggcattggt gtaccgggca ttggtgttcc gggcatcggt 1380

gtgccgggca aaggtgttcc gggcattggt gtgccgggca tcggtgtgcc aggcattggt 1440

gtgccgggca tcggtgttcc gggcaagggt gtgccgggca tcggtgtgcc aggcattggt 1500

gtaccgggca ttggtgttcc gggcatcggt gtgccgggca aaggtgttcc gggcattggt 1560

gtgccgggca tcggtgtgcc aggcattggt gtgccgggca tcggtgttcc gggcaagggt 1620

gtgccgggca tcggtgtgcc aggcattggt gtaccgggca ttggtgttcc gggcatcggt 1680

gtgccgggca aaggtgttcc gggcattggt gtgccgggca tcggtgtgcc aggcattggt 1740

gtgccgggca tcggtgttcc gggcaagggt gtgccgggca tcggtgtgcc aggcattggt 1800

gtaccgggca ttggtgttcc gggcatcggt gtgccgggca aaggtgttcc gggcattggt 1860

gtgccgggca tcggtgtgcc aggcattggt gtgccgggca tcggtgttcc gggcaagggt 1920

gtgccgggca tcggtgtgcc aggcattggt gtaccgggca ttggtgttcc gggcatcggt 1980

gtgccgggca aaggtgttcc gggcattggt gtgccgggca tcggtgtgcc aggcattggt 2040

gtgccgggca tcggtgttcc gggcaagggt gtgccgggca tcggtgtgcc aggcattggt 2100

gtaccgggca ttggtgttcc gggcatcggt gtgccgggca aaggtgttcc gggcattggt 2160

gtgccgggca tcggtgtgcc aggcattggt gtgccgggca tcggtgttcc gggcaagggt 2220

gtgccgggca tcggtgtgcc aggcattggt gtaccgggca ttggtgttcc gggcatcggt 2280

gtgccgggca aaggtgttcc gggcattggt gtgccgggca tcggtgtgcc aggcattggt 2340

gtgccgggca tcggtgttcc gggcaagggt gtgccgggca tcggtgtgcc aggcattggt 2400

gtaccgggca ttggtgttcc gggcatcggt gtgccgggca aaggtgttcc gggcattggt 2460

gtgccgggca tcggtgtgcc aggcattggt gtgccgggca tcggtgttcc gggcaagggt 2520

gtgccgggca tcggtgtgcc aggcattggt gtaccgggca ttggtgttcc gggcatcggt 2580

gtgccgggca aaggtgttcc gggcattggt gtgccgggca tcggtgtgcc aggcattggt 2640

gtgccgggca tcggtgttcc gggcaagggt gtgccgggca tcggtgtgcc aggcattggt 2700

gtaccgggca ttggtgttcc gggcatcggt gtgccgggca aaggtgttcc gggcattggt 2760

gtgccgggca tcggtgtgcc aggcattggt gtgccgggca tcggtgttcc gggcaagggt 2820

gtgccgggca tcggtgtgcc aggcattggt gtaccgggca ttggtgttcc gggcatcggt 2880

gtgccgggca aaggtgttcc gggcattggt gtgccgggca tcggtgtgcc aggcattggt 2940

gtgccgggca tcggtgttcc gggcaagggt gtgccgggca tcggtgtgcc aggcattggt 3000

gtaccgggca ttggtgttcc gggcatcggt gtgccgggca aaggtgttcc gggcattggt 3060

gtgccgggca tcggtgtgcc aggcattggt gtgccgggca tcggtgttcc gggcaagggt 3120

gtgccgggca tcggtgtgcc aggcattggt gtaccgggca ttggtgttcc gggcatcggt 3180

gtgccgggca aaggtgttcc gggcattggt gtgccgggca tcggtgtgcc aggcattggt 3240

gtgccgggca tcggtgttcc gggcaagggt gtgccgggca tcggtgtgcc aggcattggt 3300

gtaccgggca ttggtgttcc gggcatcggt gtgccgggca aaggtgttcc gggcattggt 3360

gtgccgggca tcggtgtgcc aggcattggt gtgccgggca tcggtgttcc gggcaagggt 3420

gtgccgggca tcggtgtgcc aggcattggt gtaccgggca ttggtgttcc gggcatcggt 3480

gtgccgggca aaggtgttcc gggcattggt gtgccgggca tcggtgtgcc aggcattggt 3540

gtgccgggca tcggtgttcc gggcaagggt gtgccgggca tcggtgtgcc aggcattggt 3600

<210> 37
<211> 18
<212> DNA
<213> Artificial Sequence

<220>

<223>  Sequence for a better expression of the biopolymers

<220>
<221>  CDS
<222>  (1)..(18)

<400>  37
atg gaa tca cta cta ccc                                                18
Met Glu Ser Leu Leu Pro
1               5

<210>  38
<211>  6
<212>  PRT
<213>  Artificial Sequence

<220>
<223>  Synthetic Construct

<400>  38

Met Glu Ser Leu Leu Pro
1               5

## Description

The invention relates to a recombinant biopolymer based on fusion proteins, DNA molecules encoding the fusion proteins and the biopolymer, vectors comprising the DNA molecules, host cells transformed with the vectors, and methods and kits for using them to determine or assess the activity of proteases. Specifically, the invention may be used to analyse the activity of more than one protease in a single assay and is useful for high throughput screening.

### STATE OF ART

Zymography has become a standard technique for the detection of hydrolytic enzymes, such as matrix metalloproteinases (MMPs) between others. Enzyme activities are determined based on degradation degree of a substrate. The majority of studies use the gelatine as the substrate, but there are other known substrates such as plasminogen, fibrin, casein, chondroitin sulphates and/or hyaluronan. The zymography method requires conducting electrophoresis, and therefore it is troublesome in the procedures, and time-consuming in the detection. Gelatine zymography is only detecting MMP2 and MMP9, other bands that appear in the zymogram are mainly of unknown origin, or ignored in publications. This is mainly due to the presence of a variety of possible targets in native proteins like gelatine, collagen, casein, plasminogen, fibrinogen, etc.

It could be shown, that MMPs, specifically gelatinases, are capable of degrading tumor tissues, furthermore, MMP inhibitors are able to inhibit MMP activity in human thyroid cancer. Nevertheless, it is difficult to clinically evaluate the benefit of MMP inhibitors in cancer treatments, because the *in vivo* activity of MMPs is inherently controlled by endogenous inhibitors like tissue inhibitor of metalloproteases -1 and -2 (TIMPs-1, -2). In conventional zymography the MMP-inhibitor complex is partially dissociated during the electrophoresis, and therefore the method does not allow the exact determination of the inhibitory activity of MMP inhibitors. The method is limited by the substrate that is used, information about the presence of peptidases is lacking indications about the exact cleavage site, and the type of peptidase detected is limited to the substrate.

Another drawback is the sample preparation in which tissue gets disrupted and enzymes are released, by this procedures there is a chance of colocalizing enzymes and inhibitors which would have an impact on the interpretation of the results. This drawback has been initially addressed by immunostaining method specific to peptidases, but these techniques lack the differentiation between active and inactive form of these enzymes, thus it is not feasible for peptidase inhibitor studies.

The combination of zymographic method with tissue extracts (*in-vivo* zymography (IVZ)), is a zymographic method without electrophoretic separation, where the proteolytic activity is detected by the cleavage of substrate bound fluorescent probes (gelatin, casein, or short peptides). With IVZ real-time 3D proteolytic activity in a whole organism is quantified in small animals by the release of a fluorophore. With an increasing number of accessible proteolytic fluorescence resonance energy transfer (FRET) probes, more complex 3D insights are obtained, giving new insights on the role of peptidases in the development of Zebrafish, with a so called "differential IVZ".

Particularly, MMPs are known to be secreted by cancer cells or normal cells surrounding cancers and are able to degrade extracellular matrix proteins like collagen. In this sense, type IV collagenases (MMP2 and MMP9) have been frequently reported to be accompanied with tumor invasion, metastasis and proliferation. In this sense, it is important to know the peptidase pattern, preferably when this pattern is abnormal, and can be uses as cancer biomarker.

Since the term peptidase refers to more than 13 sub-groups of proteolytic enzyme classes with about 300 entries of different peptidases (EC-list of peptidases), all involved in the degradation of proteins, there is a need in the art for methods for detection and/or accurate identification of proteases and/or protease activity in a single assay. This is a particular problem when the detection of a protease of clinical relevance has to be detected in a sample in order to diagnose a disease condition where sensitivity and specificity are important. There remains a need for a simple, rapid and low cost assay and method that provides both the high specificity and high sensitivity necessary to reliably monitor proteases activity in pathological and non-pathological conditions. Such improved assay and method therefore also find application in methods of diagnosing disease conditions where such diagnosis was not previously possible and/or reliable.

### DESCRIPTION OF THE INVENTION

The present invention discloses biologically compatible biopolymers based on fusion proteins or recombinamers with specific peptidase sensitive sequences, for the optimal detection of proteolytic enzymes. The biopolymers disclosed in the present invention are also useful for medical and veterinary diagnosis, sensors for estimation of enzyme levels, and for biomedical research applications. The biopolymers of the present invention serve as a higher specificity substitute for the active compounds that are widely used in zymography, like gelatin, fibrin, casein, plasminogen, or fluorescently labelled peptides.

Thus, the biopolymers of the invention comprise three different building blocks or areas, which can be repeated several times for higher specificity: (A) a non-staining and non-degrading area, to increase the molecular weight of the substrate; (B) a staining area, which as well allows for functionalization; (C) an area with specific proteolytic activity.

Additionally, the biopolymer of the invention can be functionalized in the building block B, with cross-linking groups for better integrity with the substrate or the matrix it is cross-linked to. The cross-link further has a stealth effect for the staining area, which is of importance for the detection of the proteolytic activities.

The non-staining and non-degrading area (A) and the staining area (B) are recombinant proteins based on elastin-mimetic proteins, elastin-like proteins or elastin-like recombinamers (ELR). These recombinants proteins are often used as biocompatible polymers, and are highly versatile as these characteristics can be modified and extended by inserting amino acids from functional domains extracted from other proteins or *de novo* natural designs (Rodríguez-Cabello JC, et al. J. Biomater. Sci. Polymer Edn,. 2007;18(3): 269-286). Said review also notes that the potential shown by ELR-based polymers has been expanded by the use of recombinant DNA technologies. This review discusses the current status of ELR polymers, with a particular emphasis on biomedical applications. It is known that ELRs comprise proteins that have an amino acid sequence and secondary structure derived from native (naturally occurring) elastin, specifically the consensus tetra (VPGG: SEQ ID NO: 17), penta (VPGXG: SEQ ID NO: 1) and hexapeptide (APGVGV: SEQ ID NO: 27). The most used sequence is the pentapeptide VPGXG (SEQ ID NO: 1), where X is any amino acid besides proline.

As used herein, the specifically exemplified elastin mimetic proteins, elastin-like proteins or elastin-like recombinamers are recombinantly produced in *Escherichia coli* or other microorganisms. The structure is described (Urry, D. W., et al. Chemical Physics Letters. 1991, v. 182, p. 101-106; Urry, D. W., et al. Biotechnological polymers: medical, pharmaceutical and industrial applications. 1993, p. 82-103), whereas the recombinant production is described in (McMillan, R. A., et al. Macromolecules. 1999, v. 32, p. 3643-3648).

Additionally, in the area with specific proteolytic activity (C) of the biopolymer of the invention, more than one proteolytic group can be included. Thus, the biopolymer of the present invention allow the design of a library of proteins for the determination and screening of the cleavage specificity of peptidases towards short peptide sequences, with the ease of the zymography method. So, the detection of all kinds of peptidases is allowed, including endo- and exo-peptidases, MMPs, serine proteases, cysteine proteases, aspartic proteases and threonine proteases, among others. Further, a novel screening method is part of the invention, using a peptide library for the parallel screening of samples, without the necessity of electrophoretic denaturation of sample. Furthermore, the method allows the determination of cleavage preferences of peptidase, without using fluorescently or antibody labelled peptides.

The biopolymer of the present invention can be used as substrate for zymographic or zymolytic techniques, in medical applications including, without limitation, in gel zymography, reverse zymography, multiple layer zymography, in vivo zymography, *in situ* zymography, and two dimensional, or isoelectric focused zymography.

The biopolymer of the present invention has important advantages with respect to other proteins, preferably native proteins, such as gelatin, fibrin, casein, plasminogen, etc., which are used for protease detection in conventional zymography. Native proteins present a variety of amino acid sequences that are possible targets for proteolytic degradation. Here an uncertainty about the resulting bands in the zymogram arises, due to a lack of information about the cleaved proteins leading to the band. This makes electrophoretic separation inevitable for characterization of the responsible peptidases, and limits the application to samples with proteases of known and distinguishable molecular weight. In other words, samples with two or more peptidases of the same size lack proper differentiation with this technique. Furthermore, the process of electrophoretic separation with the detergent Sodium Dodecyl Sulfate (SDS) limits the detection to enzymes since the proteins are denatured by the treatment with this compound. Thus, only active enzymes that refold and/or renature are detectable. In gelatin zymography the applicability is limited to the parallel detection of two MMPs differentiated by size, MMP-2 and MMP-9 and their isoforms, other bands that appear in the zymogram are mainly of unknown origin, or neglected.

Thus, in order to overcome these drawbacks in the state of the art, the present invention discloses a biopolymer based on novel arrangement of elastin mimetic peptide building blocks into one protein and additional block comprising amino acid sequences with proteolytic activity, for the more specific detection of proteases than known by the art. The biopolymer of the present invention further comprises a design that can be applied in a novel screening method for peptidases. Therefore, the biopolymer of the present invention is useful as a substitute for the native proteins used in zymography or zymolitic techniques, targeting the disadvantages of the technique with:
- a material or substrate that just possesses one proteolytic site specific to its target overcomes the need of electrophoretic separation, and thus the problems accompanied by denaturation of the sample, and
- a library of materials possessing varying proteolytic sites to screen for new peptidase targets and for the interpretation of bands detected in zymograms.

The present invention targets to open the field of zymography for the detection of potentially all peptidases, by incorporating peptidase specific sequences into the substrate. The advantage of this design is, that the block C is well known, which result in a binary output for all applied samples, if the substrate is, or is not degraded by the applied sample. The use of a library of proteolytic sequences, thus gives a sample specific pattern, which can be used to detect abnormities, which in the case of peptidases mostly related to different types of cancer or any other pathology.

A first aspect of the present invention relates to a biopolymer (hereinafter biopolymer of the invention) of general formula (I) or (II):

{[[(A¹ₐ₁BA²ₐ₂)ₙ₁C]ₘ₁(A¹ₐ₁BA²ₐ₂)ₙ₂Cᵥ]ₘ₂}ₓ (I)

B[(A¹ₐ₁A³ₐ₃A²ₐ₂)ₜ₁C]B'[(A¹ₐ₁A³ₐ₃A²ₐ₂)ₜ₂C]ₘB (II)

wherein;
A¹, A² and A³ represent a non-staining region and having each independently a SEQ ID NO: 1 (VPGXG);
B and B' represent a staining region and having each independently a sequence selected from the list consisting of: SEQ ID NO: 5 (VPGXG); SEQ ID NO: 7 (KKK); and SEQ ID NO: 8 (GGGGGKKKGGGGV), optionally B and/or B' are funcionalized with at least a cross-linked group,
C represents a proteolytic region having a sequence domain with proteolytic activity or peptidase sensitivity,
a1, a2, a3, n1, n2, m, m1, m2, t1, t2, v and x may have the same, or different, value.

In a preferred embodiment, a1, a2, a3, n1 and m have, independently, a value between 1 and 10, preferably between 1 and 5, more preferably between 1 and 3; n2 has a value of between 0 and 10, preferably between 0 and 5, more preferably between 0 and 3; v has a value between 0 and 5, preferably between 0 and 3, more preferably between 0 and 1; m1 and m2 have, independently, a value between 1 and 20, preferably between 1 and 10; and t1, t2 and x have, independently, a value between 1 and 10, preferably between 1 to 5.

Amino acid sequences A¹, A² and A³ represent a non-staining and non-degrading region (A) and preferably having each independently the SEQ ID NO: 1 (VPGXG), wherein X can be any naturally occurring amino acid besides proline, either alanine, valine, leucine, isoleucine, methionine, phenylalanine, tryptophan or glycine, preferably, alanine, valine or isoleucine.

Amino acid sequences B and B' represent a staining region (B) and preferably having each independently an amino acid sequence selected from the list consisting of: SEQ ID NO: 5 (VPGXG); wherein X is selected from any of the followings: lysine, arginine or histidine; preferably the SEQ ID NO: 5 is the SEQ ID NO: 6 (VPGKG), SEQ ID NO: 7 (KKK); and SEQ ID NO: 8 (GGGGGKKKGGGGV).

Amino acid sequences C represents the proteolytic region (C) which comprises domains with proteolytic activity, thereby allowing the biopolymer of this invention to be an appropriate substrate for detection and/or identification of all types of peptidases, preferably by zymolytic and/or zymographic techniques.

The biopolymer of the invention opens new fields of application, methods for screening and detection of peptidases and peptidase activity, by keeping the non-staining and non-degrading region (A) and the staining region (B) equal and introduce differences in the proteolytic region (C). This enables for a binary output if the region (C) is or is not a substrate to the detected hydrolase. The uncertainties about the exact cleavage site, that accompanies the use of natural proteins (gelatin, plasminogen, fibrin, casein, etc.) with their variety of possible targets is bypassed. The exposition to any proteolytic sequence, incorporated into the structure of the biopolymer of the invention allows for high throughput screening of samples in denaturing conditions or in non-denaturing conditions, preferably in non-denaturing conditions, without the use of detergents and electrophoretic separation.

A preferred embodiment of the invention refers to the biopolymer where A¹, A² and A³ have, each independently, a sequence selected from the list consisting of: SEQ ID NO: 2 (VPGIG), SEQ ID NO: 3 (VPGVG), and SEQ ID NO: 4 (VPGEG).

In another preferred embodiment of the invention refers to the biopolymer where B and B' have, each independently, a sequence selected from the list consisting of: SEQ ID NO: 6 (VPGKG), SEQ ID NO: 7 (KKK), and SEQ ID NO: 8 (GGGGGKKKGGGGV).

In another preferred embodiment, C comprises a known degradable domain comprising a sequence that is a substrate to hydrolases, this is the hydrolase is able to catalyse the degradation of peptide bonds.

The term "peptidase sensitivity" refers to the sensitivity to the hydrolysis of any peptide bond by peptidases, proteinases, and proteases. Alternatively, the term "proteolytic activity" refers to the activity of a protein for the hydrolysis of any peptide bond by peptidases, proteinases, and proteases.

In a more preferred embodiment, the sequence domain with proteolytic activity is selected from any of the mentioned domains of the peptidases selected from the list consisting of: aminopeptidases (EC 3.4.11), dipeptidases (EC 3.4.13), dipeptidyl-peptidases (EC 3.4.14), peptidyl-dipeptidases (EC 3.4.15), serine-type carboxypeptidases (EC 3.4.16), metallocarboxypeptidases (EC 3.4.17), cysteine-type carboxypeptidases (EC 3.4.18), omega peptidases (EC 3.4.19), serine endopeptidases (EC 3.4.21), cysteine endopeptidases (EC 3.4.22), aspartic endopeptidases (EC 3.4.23), metalloendopeptidases (EC 3.4.24), threonine endopeptidases (EC 3.4.25), and other not yet classified endopeptidases (EC 3.4.99).

In further preferred embodiment, the peptidase sensitivity comprises sensitivity towards oxido-reductases, transferases, hydrolases, lyases, isomerases, and/or ligases. Additionally, the term peptidase sensitivity can comprise the hydrolysis of the substrate by the same or similar to a chymotrypsin, a trypsin, an elastase, a kallikrein and/or a subtilisin hydrolysis, or a combination thereof.

In another preferred embodiment, the sequence domain with proteolytic activity belongs to the proteases selected from the list consisting of MMPs (E.C.3.4.24) and/or serine proteases (E.C.3.4.21).

In a more preferred embodiment the MMPs are selected from the list consisting of: e.g., collagenases such as MMP-1, MMP-8, or MMP-13, gelatinases such as MMP-2 or MMP-9, stromelysin-type such as MMP-3, MMP-10, MMP-11, membrane-type (MT-MMPs) such as MMP-14, MMP-16, or MMP-17, and/or matrilysin-type such as MMP-7. However, it is becoming increasingly clear that these divisions are somewhat artificial as there are a number of MMPs that do not fit into any of the traditional groups. In this sense the complete group of MMPs known is preferably MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-26, MMP-27, MMP-28, Membrane-Type MMPs and/or any combinations thereof.

In a more preferred embodiment the serine proteases are selected from the list consisting of: thrombin, tissue-type plasminogen activator (t-PA), urokinase-type plasminogen activator (u-PA), plasmin, trypsin, chymotrypsin, enterokinase, pepsin, kallikrein, cathepsin B, cathepsin H, cathepsin L, cathepsin S, cathepsin D, cathepsin K, elastase and/or any combinations thereof.

In a more preferred embodiment, the sequence domain with proteolytic activity C is selected from the list consisting of: SEQ ID NO: 9, SEQ ID NO: 10, SEQ ID NO: 11, SEQ ID NO: 12, SEQ ID NO: 13, SEQ ID NO: 14, SEQ ID NO: 15 and SEQ ID NO: 16.

In a more preferred embodiment, the SEQ ID NO: 9 belong to the elastase specific proteolytic domain; the SEQ ID NO: 10 and SEQ ID NO: 11 belong to the u-PA specific proteolytic domain, the SEQ ID NO: 12 and SEQ ID NO: 13 belong to the t-PA specific proteolytic domain, the SEQ ID NO: 14 belong to the MMP-2 specific proteolytic domain and the SEQ ID NO: 15 and SEQ ID NO: 16 belong to integrin specific binding domains.

In a more preferred embodiment, the biopolymer of the invention it is characterized in that wherein C has a sequence selected from the list consisting of: SEQ ID NO: 9, SEQ ID NO: 10, SEQ ID NO: 11, SEQ ID NO: 12, SEQ ID NO: 13, SEQ ID NO: 14, SEQ ID NO: 15 and SEQ ID NO: 16, preferably A¹, A² and A³ have each independently a sequence selected from the list consisting of: SEQ ID NO: 2, SEQ ID NO: 3, and SEQ ID NO: 4; and/or B and B' have each independently a sequence selected from the list consisting of: SEQ ID NO: 6, SEQ ID NO: 7 and SEQ ID NO: 8.

In a more preferred embodiment of the invention refers to the biopolymer wherein n2 and v has a value of 0 and m2 and x have a value of 1 in the biopolymer of formula (I), and the biopolymer has the formula (Ia):

[(A¹ₐ₁BA²ₐ₂)ₙ₁C]ₘ₁ (Ia)

wherein A¹, B, A², C, a1, a2, n1 and m1 are defined as mentioned above for the biopolymer of formula (I), preferably A¹ and A² are the same.

In a more preferred embodiment of the biopolymer of formula (Ia), A¹ and A² have the SEQ ID NO: 2, and/or B has the SEQ ID NO: 6. In a more preferred embodiment of the biopolymer of formula (Ia), C has the SEQ ID NO: 9.

In another preferred embodiment of the biopolymer of formula (Ia), a1, a2 and n1 have, independently, a value of between 1 and 5, preferably 2; and m1 has a value of between 1 and 15, preferably 10. More preferably, the biopolymer of formula (Ia) has de SEQ ID NO: 18, which is encoded by the nucleotide sequence SEQ ID NO: 28.

In a more preferred embodiment of the invention refers to the biopolymer wherein v has a value of 0, x has a value of 1 and m1 has a value of 1 in the biopolymer of formula (I), and the biopolymer has the formula (Ib):

[(A¹ₐ₁BA²ₐ₂)ₙ₁C(A¹ₐ₁BA²ₐ₂)ₙ₂]ₘ₂ (Ib)

wherein A¹, B, A², C, a1, a2, n1, n2 and m2 are defined as mentioned above for the biopolymer of formula (I), preferably A¹ and A² are the same.

In a more preferred embodiment of the biopolymer of formula (Ib), A¹ and A² have the SEQ ID NO: 2, and/or B has the SEQ ID NO: 6. In a more preferred embodiment of the biopolymer of formula (lb), C is a sequence selected from the list consisting of: SEQ ID NO: 10, SEQ ID NO: 11, SEQ ID NO: 12 and SEQ ID NO: 13.

In another preferred embodiment of the biopolymer of formula (lb), a1, a2, n1 and n2 have, independently, a value of between 1 and 5, preferably 2; and m2 has a value of between 2 and 8, preferably 4. More preferably, the biopolymer of formula (Ib) is selected from the list consisting of: SEQ ID NO: 19, SEQ ID NO: 20, SEQ ID NO: 21 and SEQ ID NO: 22, which are encoded by the nucleotide sequence SEQ ID NO: 29, SEQ ID NO: 30, SEQ ID NO: 31 and SEQ ID NO: 32, respectively.

In a more preferred embodiment of the invention refers to the biopolymer wherein x has a value of 1 in the biopolymer of formula (I), and the biopolymer has the formula (Ic):

[[(A¹ₐ₁BA²ₐ₂)ₙ₁C]ₘ₁(A¹ₐ₁BA²ₐ₂)ₙ₂Cᵥ]ₘ₂ (Ic)

wherein A¹, B, A², C, a1, a2, n1, m1, n2, v and m2 are defined as mentioned above for the biopolymer of formula (I); preferably A¹ and A² are the same, and more preferably n1, m1, n2, and v have a value of 1.

In a more preferred embodiment of the biopolymer of formula (Ic), each C is independently a sequence selected from the list consisting of: SEQ ID NO: 9 and SEQ ID NO: 16, A¹ and A² have the SEQ ID NO: 2, and/or B has the SEQ ID NO: 6.

In another preferred embodiment of the biopolymer of formula (Ic), a1 and a2 have, independently, a value of between 1 and 5, preferably 2, and m2 has a value of between 5 and 15, preferably 10. More preferably, the biopolymer of formula (Ic) is the SEQ ID NO: 25, which is encoded by the nucleotide sequence SEQ ID NO: 35.

In a more preferred embodiment of the invention refers to the biopolymer wherein v have a value of 0 in the biopolymer of formula (I), and the biopolymer has the formula (Id):

{[[(A¹ₐ₁BA²ₐ₂)ₙ₁C]ₘ₁(A¹ₐ₁BA²ₐ₂)ₙ₂]ₘ₂}ₓ (Id)

wherein A¹, B, A², C, a1, a2, n1, m1, n2, m2 and x are defined as mentioned above for the biopolymer of formula (I), preferably A¹ and A² are the same.

In a more preferred embodiment of the biopolymer of formula (Id), A¹ and A² have the SEQ ID NO: 2, and/or B has the SEQ ID NO: 6. In a more preferred embodiment of the biopolymer of formula (Id), C is the SEQ ID NO: 15.

In another preferred embodiment of the biopolymer of formula (Id), a1, a2, n1, n2, m1, m2 and x have, independently, a value of between of 1 and 5. More preferably, the biopolymer of formula (Id) is the SEQ ID NO: 24, which is encoded by the nucleotide sequence SEQ ID NO: 34.

In another preferred embodiment of the biopolymer of the invention, B has the SEQ ID NO: 7 and B' has the SEQ ID NO: 8, in the biopolymer of formula (II).

In another preferred embodiment, A¹ and A² are the same and have the SEQ ID NO: 3 and A³ has the SEQ ID NO: 4, in the biopolymer of formula (II).

In another preferred embodiment, C has the SEQ ID NO: 14, in the biopolymer of formula (II).

In another preferred embodiment, a1, a2, a3 and m, have, independently, a value of between 1 and 5; and t1 and t2 have, independently, a value of between 3 and 8, in the biopolymer of formula (II).

In another preferred embodiment, the biopolymer of formula (II) has the SEQ ID NO: 23, which is encoded by the nucleotide sequence SEQ ID NO: 33.

In another preferred embodiment, the biopolymer of the invention can be further functionalized in the amino acid building block (B and/or B'), and more preferably on the reactive amino acids displaying free amines, in particular the amines of lysine, histidine or arginine by covalent binding on the building block B and/or B'. The term "functionalized" refers to a modification of an existing molecular block, segment, group, or biopolymer, to generate or to introduce a new reactive that is capable of undergoing reaction with another functional group to form a covalent bond. For example, carboxylic acid groups can be functionalized by reaction with a carbodiimide and an imide reagent using known procedures to provide a new reactive functional group in the form of an imide group substituting for the hydrogen in the hydroxyl group of the carboxyl function. The biopolymer of the invention can be functionalized with cross-linking groups for better integrity with the substrate or matrix it is cross-linked to. The cross-linked further has a stealth effect for the staining area (building block B and/or B'), which is of importance for the detection of the proteolytic activities. In a preferred embodiment, the cross-linkers or functional groups are selected from the list consisting of acrylamide, acrylonitrile, N-vinyl pyrrolidone, a methacrylamide, acryloyloxy group, methacryl, dienyl, sorbyl, styryl, acrylamide, a vinyl or acetylene group, a biotin, avidin or other complementary complexating or coordinating groups.

The functionalization of the block B and/or B' of the biopolymer of the present invention allows a better integrity with the substrate, polymer, protein, hydrogel, etc., it is cross-linked to.

In accordance with the structures described above that lead to the biopolymers of the invention, the amino acid sequences (the term "peptides" may be used interchangeably to refer to the amino acid sequences) A¹, A², A3, B, B', and C may be joined by a covalent bond or any other type of bond that leads to a structure which maintains the properties of the biopolymers of the present invention. Said bond may be selected from, but is not limited to, the list comprising hydrogen bonds, ion pairing, hydrophobic association or inclusion complex formation.

Another aspect of the present invention refers to a nucleic acid comprising a nucleotide sequence that encodes for the amino acid sequence of any biopolymer of the invention. In a more preferred embodiment the nucleotide sequence encoding for the biopolymer comprising the SEQ ID NO: 18, is the nucleotide sequence comprises the SEQ ID NO: 28. In a more preferred embodiment the nucleotide sequence encoding for the biopolymer comprising the SEQ ID NO: 19, is the nucleotide sequence comprises the SEQ ID NO: 29. In a more preferred embodiment the nucleotide sequence encoding for the biopolymer comprising the SEQ ID NO: 20, is the nucleotide sequence comprises the SEQ ID NO: 30. In a more preferred embodiment the nucleotide sequence encoding for the biopolymer comprising the SEQ ID NO: 21, is the nucleotide sequence comprises the SEQ ID NO: 31. In a more preferred embodiment the nucleotide sequence encoding for the biopolymer comprising the SEQ ID NO: 22, is the nucleotide sequence comprises the SEQ ID NO: 32. In a more preferred embodiment the nucleotide sequence encoding for the biopolymer comprising the SEQ ID NO: 23, is the nucleotide sequence comprises the SEQ ID NO: 33. In a more preferred embodiment the nucleotide sequence encoding for the biopolymer comprising the SEQ ID NO: 24, is the nucleotide sequence comprises the SEQ ID NO: 34. In a more preferred embodiment the nucleotide sequence encoding for the biopolymer comprising the SEQ ID NO: 25, is the nucleotide sequence comprises the SEQ ID NO: 35. In a more preferred embodiment the nucleotide sequence encoding for the biopolymer comprising the SEQ ID NO: 26, is the nucleotide sequence comprises the SEQ ID NO: 36.

The nucleic acid (hereinafter nucleic acid of the invention) includes nucleic acid sequences whose transcription product, messenger RNA (mRNA), codes for the same amino acid sequence (hereinafter amino acid sequence of the invention). Variant degenerate sequences obtained from the nucleotide sequences of the invention whose product is a biopolymer with the same characteristics as the biopolymer of the invention, and also with the same functionalities, are also included. Nucleotide sequences that encode for amino acid sequences that contain modifications at their N-terminal end, C-terminal end and/or an internal amino acid position such that the function of the resulting biopolymer is the same as that resulting from translation of the mRNA sequence transcribed from the nucleotide sequence of the invention are also included. The amino acid sequence may be coded by any nucleotide sequence that leads to any of the amino acid sequences of the invention. As the genetic code is degenerate, a single amino acid may be coded by different codons (triplets), therefore the same amino acid sequence may be coded by different nucleotide sequences.

The nucleic acid of the present invention may have a nucleotide sequence that serves as a transcription initiation sequence bound at its 5' end. Likewise, the nucleic acid of the present invention may have a transcription termination sequence such as, but not limited to, the sequence GTATGA bound to its 3' end.

A further aspect of the present invention refers to an expression vector comprising the nucleic acid of the invention.

The term "expression vector" (hereinafter vector of the invention) refers to a DNA fragment that is able to replicate itself in a certain host and, as the term suggests, may serve as a vehicle for multiplying another DNA fragment (insert) fused to it. Insert refers to a DNA fragment that is fused to the vector; in the case of the present invention, the vector may comprise any of the nucleotide sequences that code for any of the biopolymers of the invention, fused to it, that may replicate itself in an appropriate host. The vectors may be plasmids, cosmids, bacteriophages or viral vectors, without excluding any other type of vector that corresponds to the given definition of a vector.

Another aspect of the present invention refers to an isolated cell transfected with the vector of the invention. Such cells will hereinafter be referred to as "cells of the invention".

The term cell, as used in the present invention, refers to a prokaryotic or eukaryotic cell. The cell may be a bacteria capable of replicating a transformed external DNA, such as any of the strains of the species *Escherichia coli,* or a bacteria capable of transferring the DNA of interest to the interior of a plant, such as *Agrobacterium tumefaciens.*

The term "transfection" refers to the introduction of external genetic material into cell using plasmids, viral vectors (the term transduction is also used in this case) or other transfer tools. For non-viral methods, the term transfection is used to refer to eukaryotic mammalian cells, whereas the term transformation is preferred to describe the non-viral transfer of genetic material in bacteria and non-animal eukaryotic cells such as fungi, alga or plants.

In a further aspect, the present invention refers to a composition comprising the biopolymer, the nucleic acid encoding for the biopolymer, the expression vector or the cell of the present invention, and another additional ingredients.

In another further aspect, the present invention refers to the use of the biopolymer, of the nucleic acid, of the expression vector, of the cell, or of the composition of the present invention, for the manufacture of a substrate for zymography or zymolytic techniques.

In another further aspect, the present invention refers to the use of the biopolymer, of the nucleic acid, of the expression vector, of the cell, or of the composition of the present invention, for the *in vitro* detection and/or identification of proteolytic enzymes.

In another further aspect, the present invention refers to the use of the biopolymer, of the nucleic acid, of the expression vector, of the cell, or of the composition of the present invention, for the *in vitro* detection and/or identification of compounds having peptidase inhibition activity. Additionally, the biopolymer, the nucleic acid, the expression vector, the cell, or the composition of the present invention, are also been useful for the analysis of the specificity or competitiveness of the inhibitors detected and/or identified.

In another aspect, the present invention refers to an *in vitro* screening method for identifying and/or detecting compounds that have peptidase inhibition activity, comprising:
a) contacting a compound to be tested as inhibitor for peptidases with a peptidase-containing sample and with the biopolymer of the present invention comprising a C block or region that is substrate to the peptidase tested, and
b) measuring the level of interaction of the compound tested with the biopolymer, wherein a reduction in the interaction or in the degradation kinetics in the presence of the test compound compared to the absence of the test compound indicates that the test compound is an inhibitor of the peptidase.

An array or kit comprising the biopolymer of the present invention which comprises different proteolytic groups is incubated with a protease-containing sample, and characterized by intensity after staining and destaining. The method gives a sample specific pattern of degraded and non-degraded substrates.

A further aspect of the present invention refers to a method for obtaining the biopolymer of the present invention that comprises:
(a) inserting the nucleic acid of the invention into an expression vector,
(b) transfecting a cell with the expression vector obtained according to section (a),
(c) selecting the transfected cell according to section (b) that comprises the nucleic acid of the invention,
(d) expressing the nucleic acid in the cell according to section (c) and
(e) purifying the biopolymer produced according to section (d).

The use of recombinant DNA technology by cloning a nucleic acid sequence that encodes for the amino acid sequence of the biopolymer of the present invention in a vector that is able to express said sequence. This approach makes use of the replication, transcription and translation machinery of the organisms to produce the biopolymers. The biocompatibility of biopolymers based on ELP (Elastin-Like Polypeptide) polypeptide sequences is a useful characteristic for the development of systems that function in contact with living tissue or specific fluids. The immune system cannot distinguish ELPs from natural elastin, therefore their biocompatibility can be considered to be extreme.

The degree of compositional complexity imposed by the need for a multifunctional design cannot be achieved using standard macromolecular synthesis techniques. The biopolymer is obtained as a recombinant protein in genetically modified microorganisms or plants using modified molecular biological and biotechnological techniques.

The nucleotide sequence that encodes for the amino acid sequence of the biopolymer of the present invention is inserted into a previously defined expression vector.

Cell transfection, as defined in a previous paragraph, is undertaken using known techniques in the state of the art, for example, but not limited to, electroporation, biolistics, chemical transformation, natural competence, *Agrobacterium tumefaciens* or any other technique that allows the incorporation of the nucleic acids of the invention into the DNA of the host cell, whether it be genomic, chloroplastic or mitochondrial.

Expression of the nucleic acid in the cell of the invention leads to a biopolymer that can be purified using known techniques in the state of the art.

A further aspect of the present invention refers to a method for *in vitro* detection and/or identification of proteolytic enzymes in an isolated biological sample, comprising the following steps:
a) mixing the biological sample comprising proteolytic enzymes and the biopolymer of the invention to obtain a hydrogel,
b) allowing the biological sample to stably bind to the biopolymer of the invention to form a detectable complex and
c) detecting the detectable complex.

In a preferred embodiment, the hydrogel obtained in step (a) forms a matrix, wherein the matrix has similar physical properties as for example acrylamide gels.

In another preferred embodiment, step (a) could be in denaturing conditions. In a more preferred embodiment, the denaturing condition is in the presence of sodium dodecyl sulfate (SDS).

In a more preferred embodiment, the binding of step (b) are performed in adequate conditions selected from the list consisting of: temperature, pH, sal and ions concentrations and incubation time.

In a more preferred embodiment, the detection and identification of the proteolytic enzyme of step (c) is performed by colorimetric methods, absorption, or fluorescence. More preferably, the detection and identification of the proteolytic enzyme of step (c) is performed by colorimetric methods.

In a preferred embodiment, the method of the present invention is performed in non-denaturing conditions.

In another preferred embodiment the method of the present invention is performed without fluorescence staining.

Detection of proteolytic enzymes, quantity, or activity is thus useful as a diagnostic/prognostic marker for the presence or likelihood of disease. In addition, detection of proteolytic activity (or the inhibition thereof) is useful in screening for protease inhibitor therapeutics for the treatment of a number of pathologies.

Another aspect of the present invention refers to a kit comprising at least one of the biopolymers, the nucleic acid, the expression vector, the cell, or the composition of the present invention, optionally, comprising any combinations thereof. Furthermore, the kit of the invention also comprises additional reagents and instructions to perform the methods disclosed herein.

Another aspect of the present invention refers to the use of the kit of the invention for performing any one of the method disclosed herein, preferably, for *in vitro* detection and/or identification of proteolytic enzymes, for *in vitro* detection and/or identification of compounds having peptidase inhibition activity and for the analysis of the specificity or competitiveness of the inhibitors detected and/or identified.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

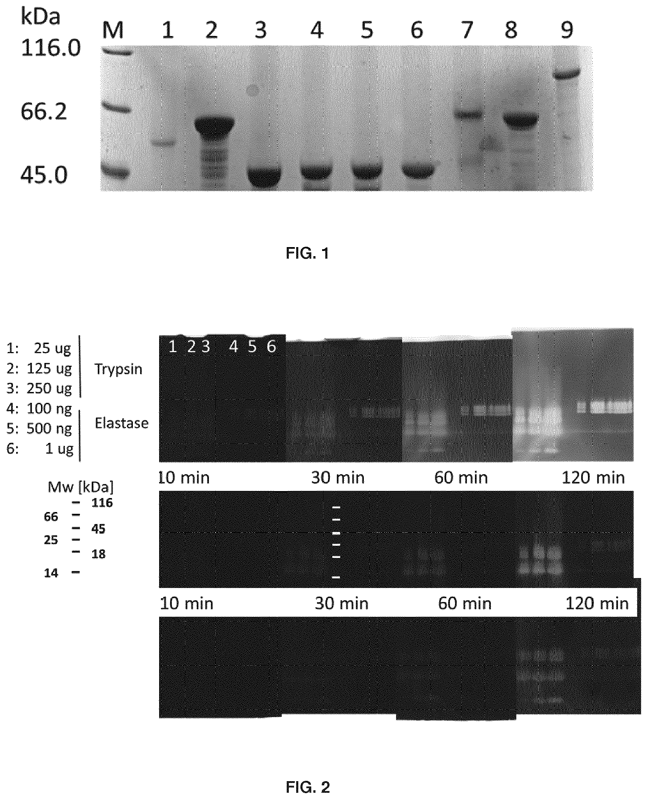

**FIG. 1****.** Photograph of polyacrylamide gel electrophoresis showing the molecular weight of biopolymers of the invention comprising the SEQ ID NO: 18 to 26. M: Marker in kDa; 1: Biopolymer comprising the SEQ ID NO: 26; 2: Biopolymer comprising the SEQ ID NO: 18; 3: Biopolymer comprising the SEQ ID NO: 19; 4: Biopolymer comprising the SEQ ID NO: 20; 5: Biopolymer comprising the SEQ ID NO: 21; 6: Biopolymer comprising the SEQ ID NO: 22; 7: Biopolymer comprising the SEQ ID NO: 23; 8: Biopolymer comprising the SEQ ID NO: 25; 9: Biopolymer comprising the SEQ ID NO: 24.
**FIG. 2****.** Photograph of polyacrylamide gel electrophoresis showing the degradation profile of the biopolymer comprising the SEQ ID NO: 18 at different enzyme and substrate concentrations and the dependence of destaining conditions. Trypsin (lane 1-3) and Elastase (lane 4-6) are charged at different concentrations (100 ng to 250 ug) into polyacrylamide gels with different concentrations of the biopolymer comprising the SEQ ID NO: 18 as substrate. Top gel: 1 mg/mL; Middle gel: 5 mg/MI; Bottom gel: 10 mg/mL. The same gel is shown at different times of destaining increasing from left (10 min) to right (120 min).

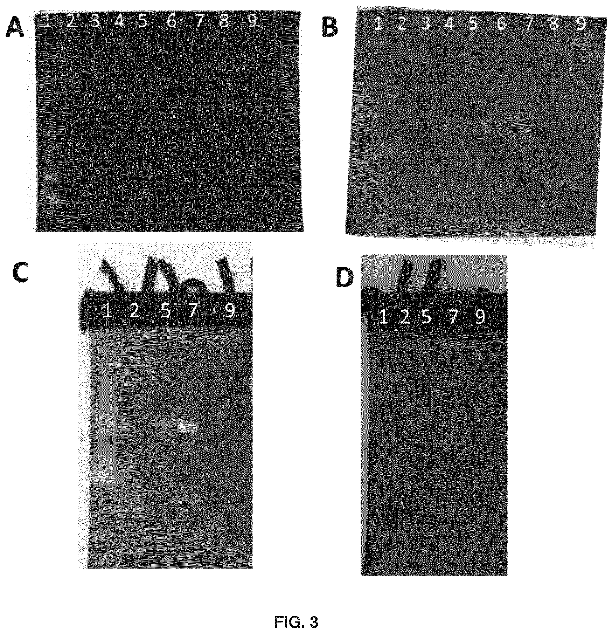

**FIG. 3****.** Photograph of polyacrylamide gel electrophoresis showing the sensitivity of the biopolymer comprising the SEQ ID NO: 25 (A), SEQ ID NO: 23 (B), gelatin (C) and SEQ ID NO: 26 (D) elastase sensitive probe to serine proteases trypsin and elastin and MMPs 2, 9 and 13. 1: Trypsin 25 ug; 2: Elastase 100 ng; 3: Marker; 4 and 5: MMP-2 50 ng and 500 ng; 6 and 7: MMP-9 50 ng and 500 ng; 8 and 9: MMP-13 50 ng and 500 ng.

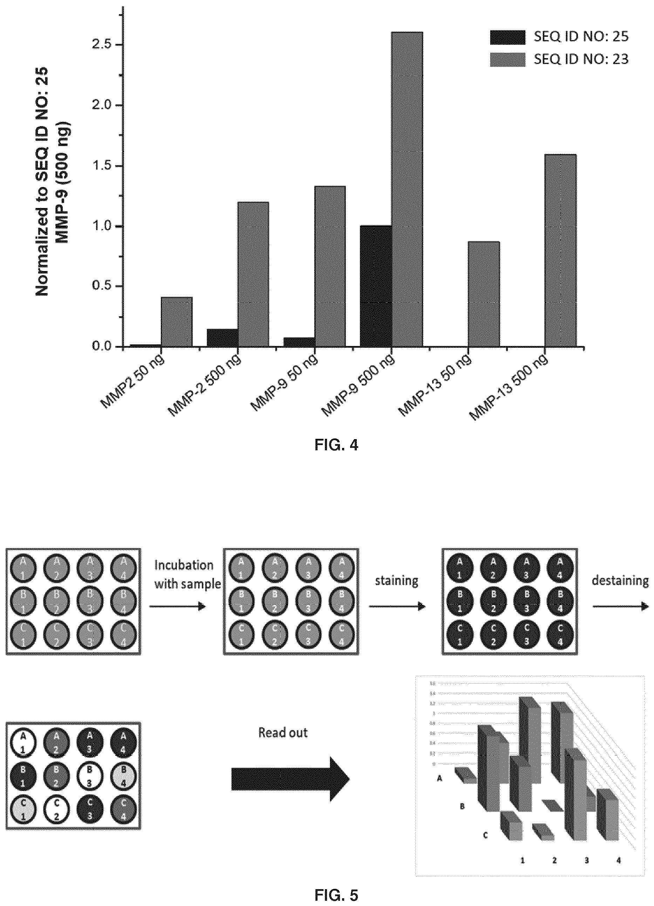

**FIG. 4****.** Densitometry of the bands from FIG. 3A (SEQ ID NO: 25) and B (SEQ ID NO: 23). For Comparison all bands where normalized to the band of MMP-9 at 500 ng (SEQ ID NO: 25).

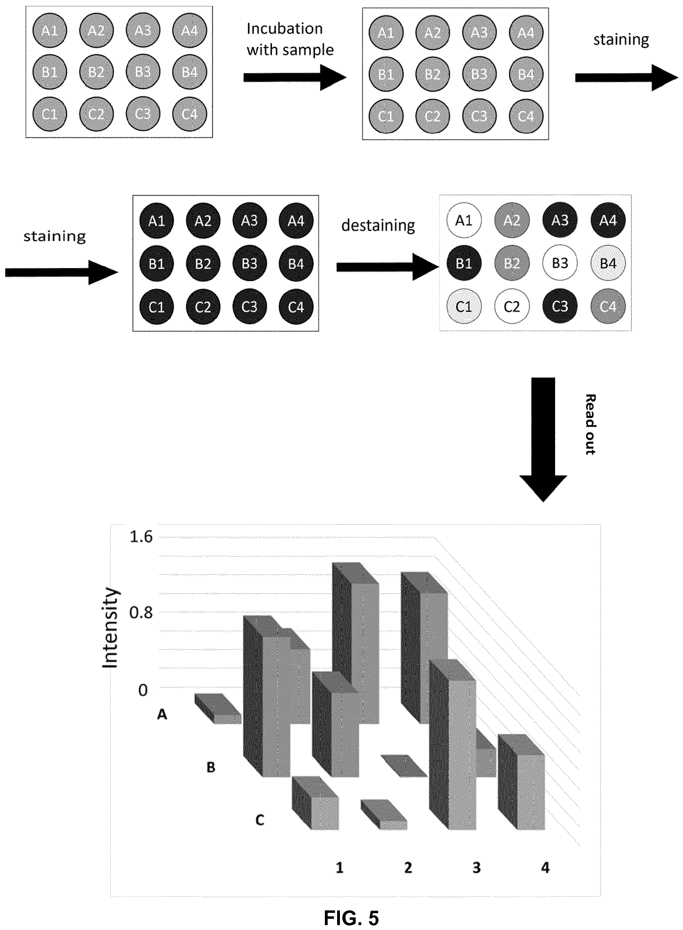

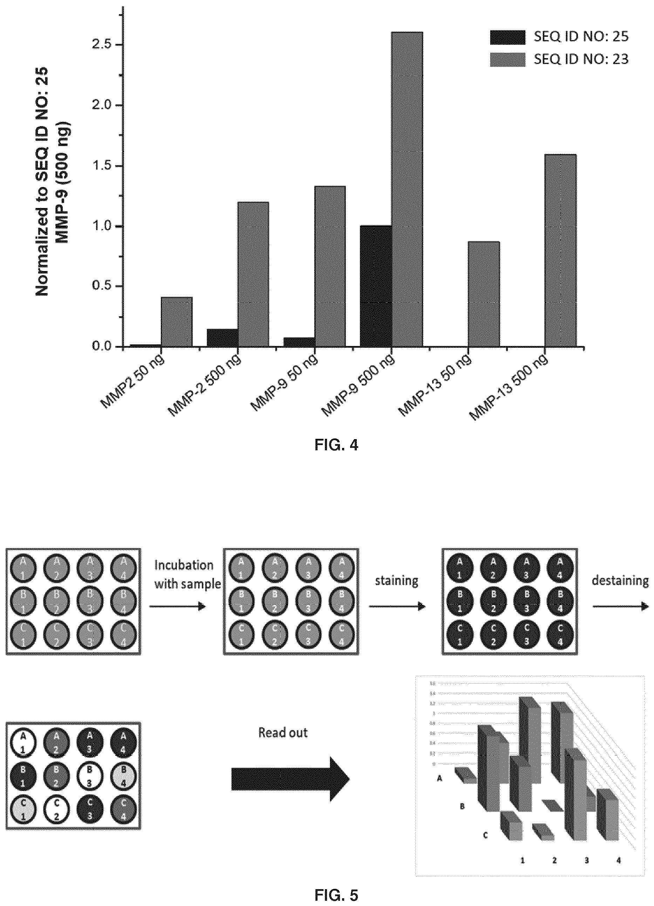

**FIG. 5****.** Scheme of the *in vitro* method for the detection and/or identification of proteolytic enzymes according to the present invention. An array comprising different biopolymers of the present invention which include different proteolytic groups is incubated with a protease-containing sample, and characterized by intensity after staining and destaining. The method gives a sample specific pattern of degraded and non-degraded substrates.

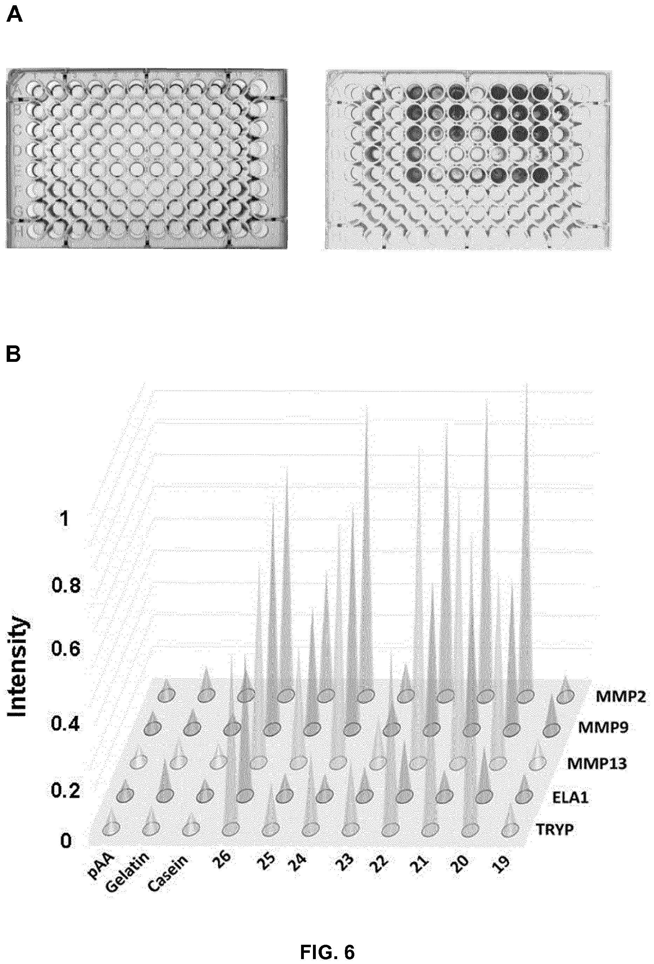

**FIG. 6****.** Patterns of proteolytic activity of the biopolymers of the invention comprising the SEQ ID NOs: 18 to 25, casein, gelatine and polyacrylamide (pAA) as substrates. All substrates were placed into 96 wellplate (A) and incubated with different enzymes, MMPs -2, -9 and 13, Elastin 1 (Ela1) and trypsin (TRYP). (B) Quantification of the intensity of absorbance at 595 nm of all samples tested in (A). polyacrylamide (pAA); 19: Biopolymer comprising the SEQ ID NO: 19; 20: Biopolymer comprising the SEQ ID NO: 20; 21: Biopolymer comprising the SEQ ID NO: 21; 22: Biopolymer comprising the SEQ ID NO: 22; 23: Biopolymer comprising the SEQ ID NO: 23; 24: Biopolymer comprising the SEQ ID NO: 24; 25: Biopolymer comprising the SEQ ID NO: 25 and 26: Biopolymer comprising the SEQ ID NO: 26.

### EXAMPLES

The invention is illustrated below by way of various trials undertaken by the inventors that describe the synthesis of the biopolymers of the present invention and uses thereof. Said examples are provided in order to be able to understand the description and are not intended to limit the present invention.

### Example 1: Design and production of the synthetic nucleotide sequence that encodes for the amino acid sequence of the biopolymers of the invention.

At the gene design stage it proved necessary to try to overcome the contradiction that exists between the use of the most common and appropriate codons that identify highly repeated amino acids and the need not to overload or even impoverish the cell translation system to the point of collapse. If heterologous prokaryotic systems are used, the problem arising from their limited use of the genetic code, which varies with species, must be taken into account. The problem arising from the creation of a gene formed by a long sequence that codes for multiple and/or small artificial fragments was also taken into account. Finally, the high recombination frequency commonly found when the exogenous DNA contains repeats of highly similar domains also had to be overcome.

This stage involved the use of automated DNA synthesizers together with recombinant DNA techniques. The production of polymers with multiple repeats necessarily involves the production of a gene that codes for them which, depending on the length of the sequence, may require the prior synthesis of a polynucleotide-based monomer that can be connected linearly in the correct direction.

The production of sufficient amounts of the monomeric gene containing the correct sequence for producing the repeat nucleotide sequences that code for the various polypeptides required culture of the appropriate clones and digestion of their plasmids. This allowed the large amount of monomer required for the controlled ligation, concatenation or concatenamerisation reaction to be obtained in a simple manner.

Although this approach may appear to be wasteful in terms of time, *in vivo* synthesis guarantees the production of the correct sequence in large amounts and also offers additional advantages, such as control of the monomeric gene prior to oligomerisation and the ability to subsequently modify the sequence by either directed mutagenesis or by the creation of copolymers prior to oligomerisation.

Polymerisation of the monomeric nucleotide sequences may be undertaken by, but is not limited to, concatenation, in other words random ligation of the monomeric blocks; the iterative/recursive method, a step-by-step technique for preparing oligomers from monomers; or by the Seamless cloning method, a definition that refers to the possibility to select a specific sequence that is translated into the desired amino acid at the cut-off point.

Concatenation allows the synthesis of polymers from oligomers by the unidirectional, linear head-to-tail attachment of the DNA segments that make up the monomer, although such attachment is only possible if the ends of the segments are single-stranded, protuberant and cohesive amongst themselves but not with themselves. Thus, the single stranded head end of the monomer will be complementary to, and will only hybridise with, the tail end of another identical monomer. These ends cannot therefore be palindromic, as currently occurs when they are generated by the restriction endonucleases routinely used in genetic engineering, where the recognition and cleavage sites occur sequentially, but must be different.

Various modifications of the concatenation technique have been developed to take into account specific requirements. Monomers with cohesive, single-stranded ends were obtained by the hybridisation of oligomers, by the joining of short oligonucleotides (linkers) and also by digestion with specific endonucleases.

The iterative/recursive method is a step-by-step technique used to prepare oligomers from monomers that allows both, the addition sequence and the number of blocks to be joined during each growth stage to be controlled, thereby allowing ad hoc biopolymer production. This method requires the creation of sequences at the ends of the segment that code for the protein-based monomer, are recognised by two different endonucleases and whose cleavage produces complementary, but not palindromic, ends. This monomeric sequence is cloned in a plasmid that serves as a gene amplification vector and provides both the following cloning vector when digested with a single enzyme and the clone insert when cleaved by two enzymes.

The *Seamless* cloning method removes the relationship between the design of the sequence for the insert that codes for the monomer and the sequences recognised by the endonucleases that generate it. This strategy is possible due to the existence of a limited number of type II restriction enzymes that recognise a specific, non-palindromic sequence that does not coincide with the cleavage site. This unique characteristic eliminates the drawbacks resulting from the need to include unusual sequences into the biopolymer in order to allow the generation of cohesive ends that allow concatenation and also means that fragments that join in a unidirectional manner can be achieved in a single digestion.

### 1.2 Biopolymers expression and purification

Once the nucleotide sequences that encode for the whole biopolymers have been created, it must be expressed. To this end, the nucleotide sequence was transferred from the corresponding cloning vector to the specialised expression vector using conventional restriction enzymes. Once it had been confirmed that this transfer had occurred correctly, a specific bacterial strain for the expression of said sequence was transformed using any of the valid techniques found in the common general knowledge. In the present invention, a strain of *Escherichia coli* suitable for expressing the exogenous genes by selecting the transforming colonies using the appropriate antibiotic was used.

Expression of the recombinant polypeptides was initiated by inoculating an isolated colony containing the corresponding recombinant vector in liquid LB medium with the eventual resistance antibiotic for the strain and the acquired resistance antibiotic with the expression vector and 1% glucose.

The bacterial culture was incubated at 37ºC, with orbital shaking at 250 rpm, for approximately 11 hours, then this culture broth was used to inoculate a fresh medium in a 1/30 ratio, preferably a 100-fold larger volume of TB medium with the same antibiotic and a concentration of 0.8% (v/v) glycerol, 0.2% (w/v) alpha-lactose and 0.05% (w/v) glucose. The mean volume in the Erlenmeyers did not exceed 20-25% of their capacity in order to ensure good oxygenation of the culture, and bacterial growth was continued under the same conditions until an optical density of around 0.6 at 600 nm had been achieved. At that point expression of the recombinant biopolymer was induced by adding isopropyl -beta-D-1-thiogalactopyranoside (IPTG) to a final concentration of 1 mM, and the resulting culture was incubated at the appropriate temperature for the time required for each experiment.

Once induction was complete, further growth and metabolism of the bacteria were inhibited by cooling to 4ºC. The cells were subsequently isolated by centrifuging for 10 min at 5000g and 4ºC, and washed twice with 100 mL/L of culture in Tris buffered saline (TBS; Tris-base 20 mM, NaCl 150 mM pH 8), the bacteria resuspended by vigorous stirring and/or pipetting and the mixture centrifuged again for 10 min at 5000g and 4ºC. The supernatant was then decanted and the sediment resuspended in 25 mL/L of culture with TE solution (Tris-base 20 mM, EDTA 1 mM pH 8) by vigorous stirring and/or pipetting. The resulting mixture was maintained at 4ºC and 10 Pg/mL of the protease inhibitor phenylmethylsulfonyl fluoride (PMSF) added.

The bacteria were lysed by sonication in a Sonicator 3000 apparatus (Misonix, New York) for 6 cycles of 3.5 minutes, each consisting of pulses of 2 seconds every 5 seconds at a power of around 100 W. The sample was maintained on ice throughout this process in order to prevent heat-induced denaturation and precipitation of the proteins. Finally, it was centrifuged for 60 minutes at 15,000g and 4ºC, with the resulting supernatant containing the total soluble fraction and the sediment the total insoluble fraction. The total soluble fraction of the bacteria was acidified to pH 3.5 with hydrochloric acid diluted in water, maintaining the sample on ice and stirring. The resulting precipitate (mainly acid proteins and DNA) was then removed by centrifugation for 20 minutes at 15,000g and 4ºC. Depending on the biopolymer concerned, the saline concentration and pH of the supernatant were adjusted.

The biopolymers were purified by taking advantage of the smart nature of ELPs and their inverse transition. Purification of the recombinant biopolymers from the soluble fraction of *E*. *coli* involves successive heating/precipitation and cooling/resuspension stages. Selective precipitation was performed by heating the sample to 70 ºC for two hours. The precipitate was then separated by centrifuging for 20 minutes at 15,000g and 40 ºC, and solubilized in 2 mL/L of type I water at 4 ºC whilst stirring for 12 hours. Each purification stage was checked by polyacrylamide electrophoresis in the presence of SDS. Once the biopolymer was considered sufficiently pure, it was dialysed against type I water and lyophilized. After the final solubilisation, the dissolved biopolymer was dialysed against type I water at 4 ºC, then lyophilised and stored at -20 ºC until use.

### Example 2: Synthesis of the Elastin-like biopolymers of the invention.

The biopolymers of SEQ ID Nos: 18, 19, 20, 21, 22, 23, 24 and 25 of the invention were designed according to the techniques described in Example 1.

The process to get from the initial DNA sequence to the final DNA sequence and its translation into a protein which will be the biopolymer of the invention will be described using SEQ ID NO: 18 as an example. A similar approach will be followed or the synthesis of the remaining biopolymers of the present invention.

Starting from the amino acid sequence of interest, the DNA sequence is translated by using the expression codons of the corresponding amino acids. The sequence is than reduced to a fragment ∼200 bp, further restriction sites for the restriction enzymes EcoRI and Earl are include to the front and the end of the sequence, enabling the extraction of the sequence of interest from the vector. For the biopolymer comprising the SEQ ID NO: 18 this resulted in a final sequence of 241 bp (sequence of interest plus restriction sites), with a total length of 204 bp, which was ordered from a company specialized in the synthesis of DNA sequences.

For the multiplication of the sequence, the sequence was introduced into a cloning vector (pDrive), which can be opened by Sapl and Earl digestion. Earl digestion opens the vector before and after the sequence of interest, to remove it from the vector and Sapl digestion opens the vector behind the sequence of interest. Introduction of the sequence into the vector is obtained by a ligation of the open pDrive together with the Ear digested sequence of interest using T4 ligase. For the introduction of consecutive sequence blocks, a ligation of a Sapl digested pDrive containing the 204 bp sequence of interest is performed together with the Earl digested sequence of interest. Increasing the concentration of the insert against the concentration of the vector lead to the introduction of multiple fragments into the vector. This resulted in sequences of the length (204)ₓ₊₁ base pairs, where x is the number of inserted sequences. This process of ligation was repeated until the final sequence of (204)₁₀ base pairs were obtained. This sequence was then removed from the cloning vector by Earl digestion and transferred into an expression vector.

Due to the design of the expression vector for a better expression of the protein of interest, the sequence MESLLP (SEQ ID NO: 38) encoded by the SEQ ID NO: 37 is added before the sequence of interest, and due to the use of Earl digestion a valine (V) is added to the end of the sequence of interest resulting in a sequence of 2061 base pairs.

The sequences at each step were validated using automated dideoxy DNA sequencing with fluorescent ddNTP and agarose gel electrophoresis.

As it has been mentioned before, a similar synthesis process as disclosed by the biopolymer comprising the SEQ ID NO: 18 is performed for obtaining the remaining biopolymers disclosed in the present invention.

**Table 1. Sequences of ELRs for Zymographic purpose.**

| **Biopolymer SEQ ID NO:** | **General formula** | **Specific formula** |
|---|---|---|
| **18** | (Ia): [(A¹ₐ₁ B A²ₐ₂)ₙ₁ C]ₘ₁ | [(A¹₂B A²₂)₂C]₁₀ |
| **19** | (Ib): [(A¹ₐ₁ B A²ₐ₂)ₙ₁ C (A¹ ₐ₁ B A²ₐ₂)ₙ₂]ₘ₂ | [(A¹₂B A²₂)₂C(A¹₂BA²₂)₂]₄ |
| **20** | | |
| **21** | | |
| **22** | | |
| **23** | (II): B [(A¹ₐ₁ A³ₐ₃ A²ₐ₂)ₜ₁ C] B'[(A¹ₐ₁ A³ₐ₃ A²ₐ₂)ₜ₂ C]ₘ, B | B[(A¹₂ A³ A²₂)₅CB'(A¹₂ A³ A²₂)₅C]B |
| **24** | (Id): {[[(A¹ₐ₁BA²ₐ₂)ₙ₁C]ₘ₁(A¹ₐ₁BA²ₐ₂)ₙ₂]ₘ₂}ₓ | {(A¹ ₂BA²₂)₂[C(A¹₂BA²₂)_{2]2}}₃ |
| **25** | (Ic): [[(A¹ₐ₁ B A²ₐ₂)ₙ₁ C]ₘ₁ (A¹ₐ₁ B A²ₐ₂)ₙ₂ Cᵥ] m2 | (A¹₂BA²₂CA¹₂BA²₂C)₁₀ |
| **26** | Control (-) | (A¹₂BA²₂)₂₄ |

As a first example, the biopolymer of formula (Ia) comprising the SEQ ID NO: 18 (Table 1) comprising a proteolytic group (C) sensitive to serine proteases like elastase, is described. In this case, the non-staining region (A) comprising two pentapetides of SEQ ID NO: 2. The staining region (B) comprising one pentapeptide of SEQ ID NO: 6, and the proteolytic region (C) comprising the SEQ ID NO: 9 that are sensitive to elastase. Using the concatenation technique, the nucleotide sequence consisting of the initial sequence of 204 base pairs, encoding for the region (A¹₂BA²₂)₂C was repeated ten times. Said incorporation resulted in a sequence of 2040 base pairs that encodes for the abbreviated polypeptide: [(A¹₂BA²₂)₂C]₁₀ consisting of SEQ ID NO: 18. The biopolymer comprising the SEQ ID NO: 18 is encoded by the nucleotide sequence comprising the SEQ ID NO: 28.

The biopolymer was synthesised as described in Example 1 and 2. In order to ensure that the above monomeric sequences could be concatenated to form the complete sequence, appropriate restriction enzyme recognition sequences that, when used, leave only the GTA codon, which encodes for the amino acid valine, as the cohesive ends, were placed at its ends. This ensures that monomer chains with no skips in the reading frame or codons other than those contained in the desired sequence are formed.

The inventors also synthetized a biopolymer for use as negative control, named as Control (-) comprising the SEQ ID NO: 26 (Table 1) without any specific proteolytic sequence in the region (C). It was obtained using the same techniques described above. The non-staining region (A) comprising two pentapetides of SEQ ID NO: 2 and the staining region (B) comprising one pentapeptide of SEQ ID NO: 6. The initial sequence of 75 base pairs, encoding for the region A¹₂BA²₂ was first duplicated and the resulting duplicate (A₂BA₂)₂, further repeated twelve times, resulting in the biopolymer of SEQ ID NO: 26 comprising the formula: (A¹₂BA²₂)₂₄. The biopolymer comprising the SEQ ID NO: 26 is encoded by the nucleotide sequence comprising the SEQ ID NO: 36.

Additionally, for synthesis of the larger biopolymers of SEQ ID NO: 19 to 25, several nucleotides sequences were combined using the iterative/recursive technique and the restriction enzymes mentioned above, prior to the multiplication of the entire gene by the concatenation technique. The initial nucleotides sequences were synthesized chemically from oligonucleotides.

The biopolymers of formula (Ib) comprising the SEQ ID NOs: 19, 20, 21 or 22 (Table 1) comprising proteolytic groups sensitive to serine proteases comprising the SEQ ID NOs: 10, 11, 12, and 13 respectively, were generated by the iterative/recursive technique. The non-staining region (A) comprising three pentapetides of SEQ ID NO: 2. The staining region (B) comprising one pentapeptide of SEQ ID NO: 6 and the proteolytic region (C) comprising the proteolytic sequences SEQ ID NOs: 10, 11, 12, or 13 respectively, those are sensitive to seine proteases. Therefore, the nucleotide sequences encoding for the proteolytic sequences SEQ ID NOs: 10, 11, 12, and 13 comprises 51 base pairs where flanked by nucleotides sequences encoding for blocks of A¹₃BA²₃ each containing 150 base pairs. Before the resulting fragment of 351 base pairs (A¹₂BA²₂C(A¹₂BA²₂)₂ was repeated four times by the concatenation technique resulting in an final sequence of 1404 base pairs, which encode for the biopolymers of SEQ ID NO: 19 to 22 respectively, having the formula: [(A¹₂BA²₂)₂C(A¹₂BA²₂)₂]₄. The biopolymers comprising the SEQ ID NOs: 19 to 22 are encoded by the nucleotide sequence comprising the SEQ ID NOs: 29 to 32.

The biopolymers of formula (II) comprising the SEQ ID NO: 23 (Table 1) was designed by several combinations of the iterative/recursive technique and the concatenation technique. In the first step the nucleotide sequence encoding for the non-staining region A (A¹₂A³A²₂) of 376 base pairs is duplicated by the recursive technique. The resulting 752 base pair sequence in the next step is fused to the nucleotide sequence encoding for the proteolytic region C sensitive to MMPs 2, 9 and/or 13 and also comprising 159 base pairs. The nucleotide sequence comprising the AC block [(A¹₂A³A²₂)₅C] is organized before and after a nucleotide sequence encoding for a short staining region B (42 base pair) by two separate concatenations resulting in the nucleotide sequence encoding for [(A¹₂A³A²₂)₅C]B[(A¹₂A³A²₂)₅C] comprising 1864 base pair fragment. Further, nucleotide sequences encoding for B blocks at the beginning (B block-30 base pair) and the end (B'block-15 base pairs) are introduced by the use of a modified expression vector, resulting in the final nucleotide sequence encoding for the SEQ ID NO: 23 of 1909 base pairs. The biopolymer comprising the SEQ ID NO: 23 is encoded by the nucleotide sequence comprising the SEQ ID NO: 33.

In order to obtain the biopolymer of formula (Id) comprising the SEQ ID NO: 24 (Table 1) comprising the RGD motif which acts as receptors for cell adhesion molecules as proteolytic group (C), the fragment was duplicated using the same described techniques by joining the 150-base pair encoding for the (A¹₂BA²₂)₂-block to two fragments of the nucleotide sequence C encoding for (A¹₂BA²₂)₂ of 186 base pairs. The resulting 522 base pair fragment was then repeated three times using the concatenation technique to obtain the final nucleotide sequence encoding for the SEQ ID NO: 24 of formula: {(A¹₂BA²₂)₂[C(A¹₂BA²₂]₂}₃ comprising 1566 base pairs. The biopolymer comprising the SEQ ID NO: 24 is encoded by the nucleotide sequence comprising the SEQ ID NO: 34.

To obtain the biopolymer of formula (Ic) comprising the SEQ ID NO: 25 (Table 1) comprising the REDV motif as proteolytic group (C), a 132 base pair sequence encoding for (A¹₂BA²₂)₂C and a 129 base pair-block encoding for (A¹₂BA²₂₎₂Cv were fused using the same technique as described above. The resulting fragment of 261 base-pairs was than incorporated 10 more times by the concatenation technique to obtain the final nucleotide sequence encoding for the SEQ ID NO: 25 of formula (A¹₂BA²₂CA¹₂BA²₂C)₁₀ comprising 2610 base pairs. The biopolymer comprising the SEQ ID NO: 25 is encoded by the nucleotide sequence comprising the SEQ ID NO: 35. All sequences were incorporated into an expression vector previously modified to accept fragments produced by digestion with the described appropriate restriction enzymes incorporating a nucleotide sequence at the 5' end of the fragment and the sequence GTATGA at its 3' end. The resulting plasmid comprises a nucleotide sequence with an open reading frame of sequence base pairs + 24, which code for the amino acids.

### 2.2. Characterisation of the biopolymers of the invention.

Biopolymers of SEQ ID NOs: 18 to 25 and the control (-) biopolymer (SEQ ID NO: 26) were characterised by polyacrylamide gel electrophoresis (PAGE) in the presence of SDS, which allowed the molecular weight of the biopolymer to be estimated and its purity to be confirmed (**FIG. 1**). It can be seen from said figure that a good yield of electrophoretically pure biopolymer was obtained.

Additionally, the molecular weight for the biopolymers of the present invention SEQ ID NOs: 18 to 26 were also determined experimentally by MALDI-TOF using a Q-Star spectrometer. The obtained data are showing in Table 2.

**Table 2. Molecular weight for the biopolymers of the present invention determined by MALDI-TOF.**

| Biopolymer (SEQ ID NO:) | Molecular weight (MALDI-TOF) | Theoretic Molecular weight (Da) |
|---|---|---|
| 18 | 57401 | 57856 |
| 19 | 42085 | 46989 |
| 20 | 41417 | 46369 |
| 21 | 42075 | 46993 |
| 22 | 42140 | 46977 |
| 23 | 54220 | 51803 |
| 24 | 61302 | 60661 |
| 25 | 80503 | 80911 |
| 26 | 51623 | 51969 |

### 2.3. Modification of the biopolymer

The biopolymers of SEQ ID NOs: 18 to 26 having a molecular weight between 40 and 90 kDa were synthesized by a recombinant genetic engineering method, the details of which have been reported elsewhere (McMillan, R. A., et al. 1999, 32: 9067-9070). Lysine modification with BCN-NHS esters and/or methacrylic anhydride have been accomplished to a degree of 50%, thus 50% lysines remained with free amine groups for detection by coomassie Brilliant Blue staining.

### Example 3: Sensitivity of elastase sensitive biopolymer of SEQ ID NO: 18 to serine proteases trypsin and elastin.

Preparation of substrate gels and subsequent detection of proteolytic activity were conducted as previously described according to the methods of Heussen, et al. (Heussen, C., and Dowdle, E. B., 1980. Vol. 102, p: 196-202) by mixing the biopolymer comprising the SEQ ID NO: 18 at different concentrations (1, 5 and 10 mg/ml) into a 10% polyacrylamide gel. Preparation of biopolymer as substrate gel was carried out in Laemmli buffer system except that the amount of ammonium sulphate was doubled. Trypsin and elastase were also used at different concentrations 100 ng to 250 ug and firstly were separated by electrophoresis on a 10% polyacrylamide gel containing SDS. The electrophoresis was performed at 25° C at constant current of 40 mA. The gel was washed three times for 20 minutes in 2.5% Triton X-100, before incubation in 50 mM Tris buffer (pH 7.5) containing 5 mM Ca²⁺, 150 mM NaCl. After 40h incubation at 37° C., the gels were stained with 0.25% Coomassie blue in 5% acetic acid and 30% methanol for 0.5 hour and destained with 10% acetic acid and 40% methanol. Images of destained gels were taken at 10 min, 30, min, 1 h, and 2h. Proteolytic activities were detected by clear zones indicating the lysis of the substrate (FIG. 2).

It could be observed that already at as low concentrations as 1 mg/mL of the biopolymer comprising the SEQ ID NO: 18 clear bands of enzyme activity could be observed. Furthermore, low concentrations respond faster to the destaining and bands were clearly visible after 30 minutes. One drawback of low biopolymer concentrations might be a vanishing of the background staining under prolonged destaining <60 minutes. For longer destaining times higher concentrations of biopolymer can be used to enhance the contrast of the staining. It has to be considered, that high biopolymer concentrations also need longer incubation times for proteolysis at 37°C.

### Example 4. Sensitivity of gelatin compared to biopolymers of SEQ ID NO: 23, 25 and 26 to serine proteases trypsin and elastin and MMPs 2, 9 and 13.

Gels with the substrates gelatin, biopolymer of SEQ ID NO: 26 (negative control), biopolymer of SEQ ID NO: 25 or biopolymer of SEQ ID NO: 23 at a concentration of 1 mg/ml were prepared according to Example 2. The gels were loaded with different concentrations of MMPs 2, 9, and 13 (ranging from 100 ng to 250 ug) and with the serine proteases elastase and trypsin. The MMPs 2, 9 and 13, at the concentrations mentioned above, were first separated by electrophoresis on a 10% polyacrylamide gel containing SDS. The electrophoresis was performed at 25° C at constant current of 40 mA. The gel was washed three times for 20 minutes in 2.5% Triton X-100, before incubation in 50 mM Tris buffer (pH 7.5) containing 5 mM Ca²⁺, 150 mM NaCl, 10 uM Zn²⁺. After 40h incubation at 37° C, the gels were stained with 0.25% Coomassie blue in 5% acetic acid and 30% methanol for 0.5 hour and destained with 10% acetic acid and 40% methanol. Images of destained gels were taken at 10 min, 30, min, 1 h, and 2h. Proteolytic activities were detected by clear zones indicating the lysis of the substrate (**FIG. 3**). The example shows the potential of specific zymography for the interpretation of the obtained bands.

The results show that differences in peptidases concentrations can be measured. The resulting bands of the gel showed in **FIG: 3** were further quantified by densitometry using imageJ (**FIG. 4**). Gelatine reacts very sensitive to trypsin (**FIG. 3C**, lane 1) and the whole lane seems to be degraded with two regions of higher intensity even under incubation conditions that contain Zn²⁺ ions that are known to inhibit serine proteases. Elastase was not able to degrade gelatine under this conditions (**FIG. 3C****,** lane 2) neither did MMP-13 (**FIG. 3C**, lane 9). Recombinant MMP-2 and MMP-9 which are the known substrate for gelatine zymography led to clear bands (**FIG. 3C**, lanes 5 and 7).

The negative control (biopolymer comprising the SEQ ID NO: 26) without any proteolytic region C (**FIG. 3D**) was not degraded by the selected enzymes under the selected conditions. When the SEQ ID NO: 23 was uses as substrate, serine proteases trypsin and elastase were not detected (**FIG. 3B**, lanes 1 and 2), but MMPs -2, -9 and - 13 were detected (**FIG. 3B** lines 4 to 9 and **FIG. 4**). Regarding the biopolymer comprising the SEQ ID NO: 25 as substrate, it was note that was sensitive to both trypsin and elastase (**FIG 3A** lines 1 and 2). The trypsin signal was more concentrated in bands than in the case of gelatin degradation where the whole lane was degraded, and in contrast to gelatine, with SEQ ID NO: 25 also elastase was detected. The differences of intensity of SEQ ID NO: 18 in Example 3 and SEQ ID NO: 25 in Example 4 derive from the inhibition of serine proteases by the presented Zn²⁺ ions that are essential for the renaturation of MMPs. Therefore, even though both proteins contain the same proteolytic group SEQ ID NO: 9, a difference in serine protease sensitivity occured in the presence of bivalent ions like Zn²⁺. MMP-2 was only detected at high concentrations, whereas MMP-9 was able to degrade the substrate comprising the biopolymer of SEQ ID NO: 25 under the described conditions and MMP-13 was not detected.

MMP-13 was not detected with gelatine as substrate but it was detected when the biopolymers of the present invention (SEQ ID NO: 23 and 25) was used as substrate. This result shows that the specific design of the biopolymers of the invention allows for specific detection that is superior to the use of natural proteins since the biopolymers of the invention can be tailored to the application and furthermore, the sites of enzymatic cleavage are known.

### Example 5: Screening of zymographic activity of peptidases.

The present invention opens new fields of application for method of screening and activity detection and/or identification of peptidases. By maintaining a similar protein basis inactive towards proteases (blocks or regions A and B), where just differences in the proteolytic region (block or region C) are present, thus it gives binary information if the hydrolase can or cannot cleave the substrate. The uncertainty about the exact cleavage site that accompanies the use of natural proteins, such as gelatin, with their variety of possible targets is bypassed. The method disclosed in the present invention is schematically described in **FIG. 5****.** An array of different proteolytic groups included in different biopolymers design according to the present invention is incubated with a protease-containing sample, and characterized by intensity after staining and destaining. The method gives a sample specific pattern of degraded and non-degraded substrates.

The exposition to any proteolytic sequence, incorporated into the same recombinant protein of the biopolymer of the invention, allows for high throughput screening of samples in non-denaturing conditions, without the use of detergents and electrophoretic separation.

Gels comprising the biopolymers comprising the SEQ ID Nos: 18 to 26, gelatin or casein (1 mg/mL) were prepared as substrate in 10% acrylamide without the use of SDS. 50 uL of gel solution were jellified in a 96 well-plate. The MMP -2, -9 and -13 were added (100 ng/well) in 50 mM Tris buffer (pH 7.5) containing 5 mM Ca²⁺, 150 mM NaCl, 10 uM Zn²⁺. The elastase (100ng/well) and trypsin (500 ng/well) were added (100ng/well) in 50 mM Tris buffer (pH 7.5) containing 5 mM Ca²⁺, 150 mM NaCl. All peptidases were incubated for 16 h at 37ºC. The gels were stained with 0.25% Coomassie blue in 5% acetic acid and 30% methanol for 0.5 hour and destained with destaining solution (10% acetic acid and 40% methanol) for 10 minutes. Absorbance at 595 nm was detected and plotted (**FIG. 6**).

Results show that using eight different biopolymers according to the present invention comprising the SEQ ID NOs: 18 to 25 different patterns of proteolytic activity are obtained. Consequently, with the approach of the present invention it is possible to generate specific pattern of peptidases i.e. in biological clinical samples.

Furthermore, in the known zymographic methods, the majority of proteases is not characterized and just ordered by molecular weight. However, the biopolymer of the present invention allows a new insight in the diagnostics of proteases from clinical relevant samples. It is believe that the biopolymer of the invention will help to discover new proteases and their related cleavage patterns. Further, the use of the biopolymer of the invention also allows the correlation of more complex biological samples, based on their cleavage patterns.

## Claims

1. A biopolymer of general formula (I) or (II):
{[[(A¹ₐ₁BA²ₐ₂)ₙ₁C]ₘ₁(A¹ₐ₁BA²ₐ₂)ₙ₂Cᵥ]ₘ₂}ₓ (I)
B [(A¹ₐ₁A³ₐ₃A²ₐ₂)ₜ₁C]B'[(A¹ₐ₁A³ₐ₃A²ₐ₂)ₜ₂C]ₘB (II)
wherein,
A¹, A² and A³ represent a non-staining region and having each independently a SEQ ID NO: 1;
B and B' represent a staining region and having each independently a sequence selected from the list consisting of: SEQ ID NO: 5; SEQ ID NO: 7 and SEQ ID NO: 8, optionally B and/or B' are funcionalized with at least a cross-linked group,
C represents a proteolytic region having a sequence domain with proteolytic activity,
a1, a2 and a3 have, independently, a value of between 1 and 10,
n1 has a value of between 1 and 10,
n2 has a value of between 0 and 10,
v has a value of between 0 and 5,
m1 and m2 have, independently, a value of between 1 and 20,
m has a value of between 1 and 10,
t1 and t2 have, independently, a value of between 1 and 10, and
x has a value of between 1 and 10.

2. The biopolymer according to claim 1, wherein C has a sequence selected from the list consisting of: SEQ ID NO: 9, SEQ ID NO: 10, SEQ ID NO: 11, SEQ ID NO: 12, SEQ ID NO: 13, SEQ ID NO: 14, SEQ ID NO: 15 and SEQ ID NO: 16, preferably A¹, A² and A³ have each independently a sequence selected from the list consisting of: SEQ ID NO: 2, SEQ ID NO: 3, and SEQ ID NO: 4; and/or B and B' have each independently a sequence selected from the list consisting of: SEQ ID NO: 6, SEQ ID NO: 7 and SEQ ID NO: 8.

3. The biopolymer according to any of claims 1 to 2, wherein the biopolymer of general formula (I) is selected from:
a biopolymer of formula (Ia) when n2 and v has a value of 0 and m2 and x have a value of 1:
[(A¹ₐ₁BA²ₐ₂)ₙ₁C]ₘ₁ (Ia)
a biopolymer of formula (Ib) when v has a value of 0, x has a value of 1 and m1 has a value of 1:
[(A¹ₐ₁BA²ₐ₂)ₙ₁C(A¹ₐ₁BA²ₐ₂)ₙ₂]ₘ₂ (Ib)
a biopolymer of formula (Ic) when x has a value of 1:
[[(A¹ₐ₁BA²ₐ₂)ₙ₁C]ₘ₁(A¹ₐ₁BA²ₐ₂)ₙ₂Cᵥ]ₘ₂ (Ic)
or
a biopolymer of formula (Id) when v has a value of 0:
{[[(A¹ₐ₁BA²ₐ₂)ₙ₁C]ₘ₁(A¹ₐ₁BA²ₐ₂)ₙ₂]ₘ₂}ₓ (Id)
wherein A¹, B, A², C, a1, a2, n1, n2, m1, m2 and x are defined in any one claims 1 or 2.

4. The biopolymer according to claim 3, wherein the biopolymer is the formula (Ia) and wherein C has the SEQ ID NO: 9, a1, a2 and n1 have, independently, a value of between 1 and 5; and m1 has a value of between 1 and 15.

5. The biopolymer according to claim 3, wherein the biopolymer is the formula (Ib) and wherein C is a sequence selected from the list consisting of: SEQ ID NO: 10, SEQ ID NO: 11, SEQ ID NO: 12 and SEQ ID NO: 13, a1, a2, n1 and n2 have, independently, a value of between 1 and 5; and m2 has a value of between 2 and 8.

6. The biopolymer according to claim 3, wherein the biopolymer is the formula (Ic) and wherein each C is independently a sequence selected from the list consisting of: SEQ ID NO: 9 and SEQ ID NO: 16, a1 and a2 have, independently, a value of between 1 and 5; and m2 has a value of between 5 and 15.

7. The biopolymer according to claim 3, wherein the biopolymer is the formula (Id) and wherein C has the SEQ ID NO: 15, and a1, a2, n1, n2, m1, m2 and x have, independently, a value of between of 1 and 5.

8. The biopolymer according to any of claims 1 or 2, wherein the biopolymer is the formula (II) and wherein B has the SEQ ID NO: 7, B' has the SEQ ID NO: 8, A¹ and A² are the same and have the SEQ ID NO: 3, A³ has the SEQ ID NO: 4, and C has the SEQ ID NO: 14.

9. The biopolymer according to claim 8 wherein a1, a2, a3 and m, have, independently, a value of between 1 and 5; and t1 and t2 have, independently, a value of between 3 and 8.

10. The biopolymer according to claim 1, wherein the biopolymer the general formula (I) is selected from the list consisting of SEQ ID NO: 18, SEQ ID NO: 19, SEQ ID NO: 20, SEQ ID NO: 21, SEQ ID NO: 22, SEQ ID NO: 25 and SEQ ID NO: 24 and the biopolymer the general formula (II) has the SEQ ID NO: 23.

11. The biopolymer according to any of claims 1 to 10 wherein B and/or B' are further functionalized, preferably the functionalized group is selected from the list consisting of: acryloyloxy group, methacryl, dienyl, sorbyl, styryl, acrylamide, acrylonitrile, N-vinyl pyrrolidone, a methacrylamide, a vinyl group, an acetylene group, a biotin group, an avidin group, and any combinations thereof.

12. A nucleic acid comprising a nucleotide sequence that encodes for the amino acid sequence of the biopolymer according to any of claims 1 to 11.

13. An expression vector comprising the nucleic acid according to claim 12.

14. An isolated cell transfected with the vector according to claim 13.

15. Use of the biopolymer according to any of claims 1 to 11, of the nucleic acid according to any of claims 12, of the expression vector according to claim 13, of the cell according to claim 14, for the manufacture of a substrate for zymography technique, or for the *in vitro* detection and/or identification of proteolytic enzymes, or for identifying and/or detecting compounds that have peptidase inhibition activity.
